(19) Europäisches Patentamt
European Patent Office
Office européen des brevets

(11) EP 0 482 539 B1

(12) **EUROPEAN PATENT SPECIFICATION**

(45) Date of publication and mention of the grant of the patent: **18.12.1996 Bulletin 1996/51**

(51) Int. Cl.$^6$: **C07K 5/06**, A61K 38/00

(21) Application number: **91117889.5**

(22) Date of filing: **20.10.1991**

(54) **Peptide compounds, processes for preparation thereof and pharmaceutical composition comprising the same**

Peptidverbindungen, Verfahren zu deren Herstellung und diese enthaltende pharmazeutische Präparate

Composés peptidiques, leur préparation et composition pharmaceutique les contenant

(84) Designated Contracting States:
**AT BE CH DE DK ES FR GB GR IT LI LU NL SE**

(30) Priority: **24.10.1990 GB 9023116**

(43) Date of publication of application:
**29.04.1992 Bulletin 1992/18**

(73) Proprietor: **FUJISAWA PHARMACEUTICAL CO., LTD.**
**Osaka-shi Osaka 541 (JP)**

(72) Inventors:
- **Matsuo, Masaaki**
  **Toyonaka-shi, Osaka 560 (JP)**
- **Hagiwara, Daijiro**
  **Moriguchi-shi, Osaka 570 (JP)**
- **Miyake, Hiroshi**
  **Kyoto-shi, Kyoto 606 (JP)**

(74) Representative: **Türk, Gille, Hrabal, Leifert**
**Brucknerstrasse 20**
**40593 Düsseldorf (DE)**

(56) References cited:
**EP-A- 0 074 787**
**EP-A- 0 333 174**
**EP-A- 0 394 989**

Remarks:
The file contains technical information submitted after the application was filed and not included in this specification

EP 0 482 539 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.10/3.4

## Description

The present invention relates to peptide compounds and pharmaceutically acceptable salt thereof, which have pharmacological activities such as tachykinin antagonism, especially substance P antagonism, neurokinin A antagonism, neurokinin B antagonism, to processes for preparation thereof, to pharmaceutical composition comprising the same, and to a use of the same as a medicament. EP-A-0 394 989 discloses peptide compounds which have pharmacological activities such as tachykinin antagonism corresponding to the compounds of present claim 1 with the exception that $R^3$, which is hydrogen or hydroxy in D1, is a substituent other than hydroxy in the present application.

EP-A-0 333 174 discloses peptide compounds having tachykinin antagonism, which differ from the compounds of the present application by having a D-Trp amino acid residue in the central position in place of a hydroxyproline or didehydroproline residue.

The peptide compounds of the present invention are useful as tachykinin antagonist, especially substance P antagonist, neurokinin A antagonist or neurokinin B antagonist, useful for treating or preventing tachykinin mediated diseases, for example, respiratory diseases such as asthma, bronchitis, rhinitis, cough, expectoration; ophthalmic diseases such as conjunctivitis, vernal conjunctivitis; cutaneous diseases such as contact dermatitis, atopic dermatitis, urticaria, and other eczematoid dermatitis; inflammatory diseases such as rheumatoid arthritis, osteoarthritis; pains or aches (e.g., migraine, headache, toothache, cancerous pain, back pain; in human being or animals.

The object compounds of the present invention can be represented by the following general formula (I).

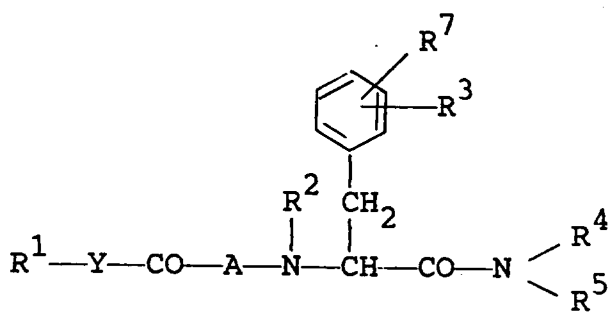

wherein

$R^1$ is $C_6$-$C_{10}$ aryl which may have one to three substituent(s) selected from halogen and $C_{1-6}$ alkoxy;
benzofuryl;
pyridyl;
or a group of the formula :

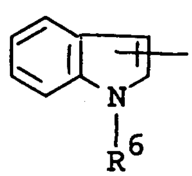

wherein $R^6$ is hydrogen; or
$C_{1-6}$ alkyl,

$R^2$ is hydrogen or $C_{1-6}$ alkyl,

$R^3$ is $C_{1-6}$ alkyl which may have one to three halogen;
amino;
acylamino;
carboxy($C_{1-6}$)alkoxy;
$C_{1-6}$ alkoxycarbonyl($C_{1-6}$)alkoxy;
halogen;
$C_{1-6}$ alkoxy; or nitro,

$R^4$ is $C_{1-6}$ alkyl,

$R^5$ is mono or di or triphenyl($C_{1-6}$)alkyl,

$R^7$ is hydrogen; or

$C_{1-6}$ alkyl;

A is hydroxyproline, and

Y is bond, $C_{1-6}$ alkylene or $C_{2-6}$ alkenylene.

According to the present invention, the peptide compounds (I) can be prepared by processes which are illustrated in the following schemes.

Process 1

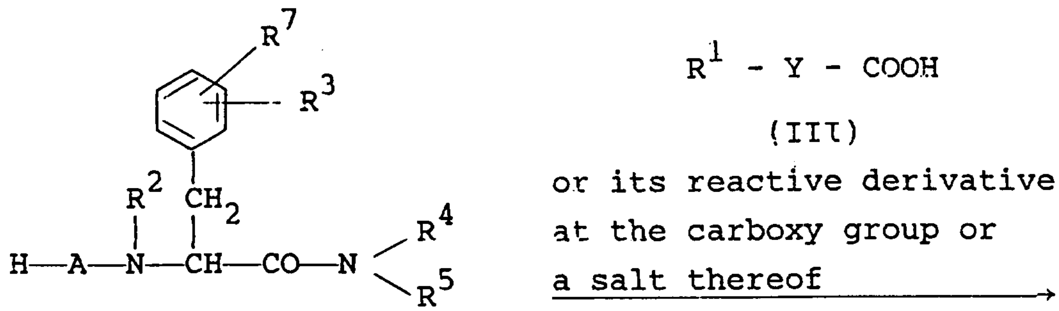


(II)

or its reactive derivative at the amino group or a salt thereof

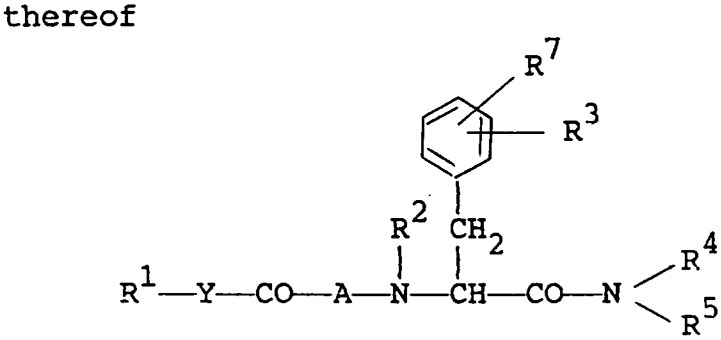

(I)

or a salt thereof

Process 2

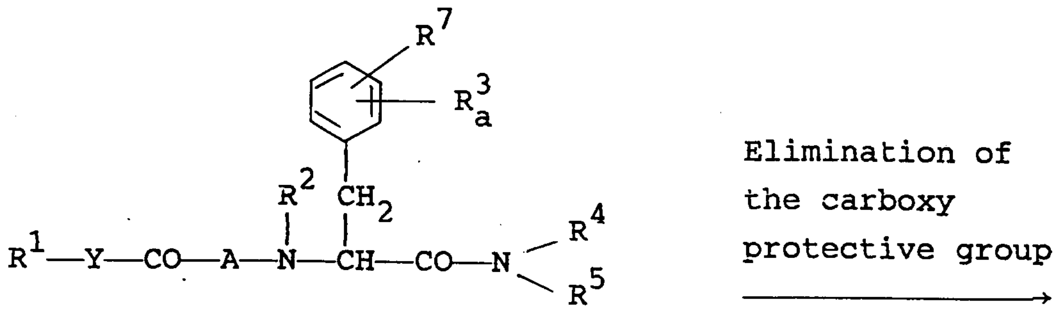


(I-p)
or a salt thereof

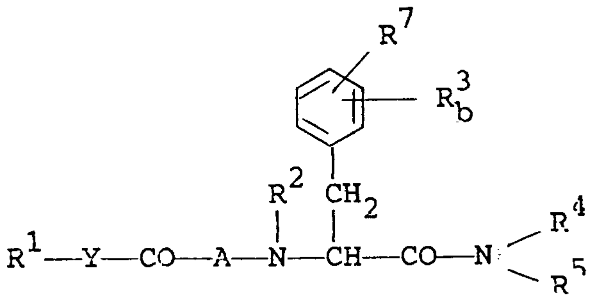

(I-q)
or a salt thereof

Process 3

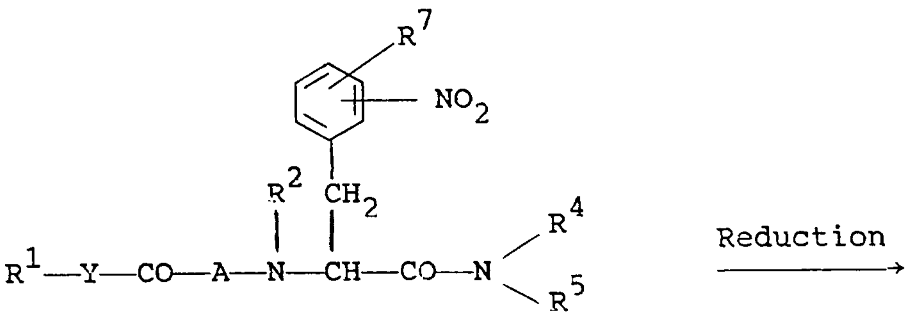


(I-r)
or a salt thereof

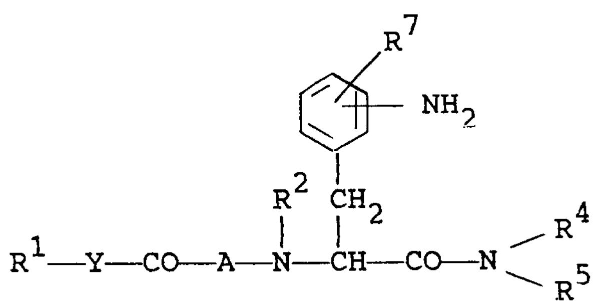

(I-s)
or a salt thereof

wherein

| | |
|---|---|
| $R^1$, $R^2$, $R^3$, $R^4$, $R^5$, $R^7$, A, X and Y are | each as defined above, |
| $R^3_a$ is | $C_{1-6}$ alkoxycarbonyl($C_{1-6}$)alkoxy, and |

$R^3_b$ is carboxy($C_{1-6}$)alkoxy.

As to the starting compounds (II) and (III), some of them are novel and can be prepared by the procedures described in the preparations and Examples mentioned later or a conventional manner.

Throughout the present specification, the amino acid, peptides, protective groups, condensing agents, etc. are indicated by the abbreviations according to the IUPAC-IUB (Commission on Biological Nomenclature) which are in common use in the field of art.

Moreover, unless otherwise indicated, the amino acids and their residues when shown by such abbreviations are meant to be L-configured compounds and residues.

Suitable pharmaceutically acceptable salts of the starting and object compound are conventional non-toxic salt and include an acid addition salt such as an organic acid salt (e.g. acetate, trifluoroacetate, maleate, tartrate, methanesulfonate, benzenesulfonate, formate, toluenesulfonate), an inorganic acid salt (e.g. hydrochloride, hydrobromide, hydroiodide, sulfate, nitrate, phosphate), or a salt with an amino acid (e.g. arginine, aspartic acid, glutamic acid), or a metal salt such as an alkali metal salt (e.g. sodium salt, potassium salt) and an alkaline earth metal salt (e.g. calcium salt, magnesium salt), an ammonium salt, an organic base salt (e.g. trimethylamine salt, triethylamine salt, pyridine salt, picoline salt, dicyclohexylamine salt, N,N'-dibenzylethylenediamine salt,).

In the above and subsequent descriptions of the present specification, suitable examples and illustrations of the various definitions which the present invention include within the scope thereof are explained in detail as follows.

The term "$C_{1-6}$" is intended to mean 1 to 6, preferably 1 to 4 carbon atom(s), unless otherwise indicated.

Suitable "$C_{1-6}$ alkyl" may include a straight or branched one such as methyl, ethyl, propyl, isopropyl, butyl, isobutyl, tert-butyl, pentyl, hexyl, in which the most preferred one is methyl.

Suitable "$C_{1-6}$ alkylene" is one having 1 to 6 carbon atom(s) and may include methylene, ethylene, trimethylene, propylene, tetramethylene, methyltrimethylene, hexamethylene, in which the preferred one is methylene, ethylene or trimethylene.

Suitable "$C_{2-6}$ alkenylene" is one having 2 to 6 carbon atom(s) and may include vinylene, propenylene, in which the preferred one is vinylene.

Suitable "carboxy($C_{1-6}$)alkoxy" may include carboxymethyl, carboxyethoxy, carboxypropoxy.

Particularly, the preferred embodiments of $R^1$, $R^2$, $R^3$, $R^4$, $R^5$, $R^7$, A and Y are as follows.

$R^1$ is phenyl and naphthyl, which may have one to three halogen or $C_{1-6}$ alkoxy (e.g. phenyl, difluorophenyl, dimethoxyphenyl);
benzofuryl;
pyridyl;
or a group of the formula :

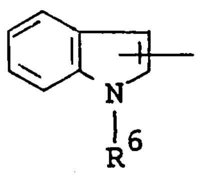

wherein $R^6$ is hydrogen; or
$C_{1-6}$ alkyl (e.g. methyl);

$R^2$ is hydrogen; or
$C_{1-6}$ alkyl (e.g. methyl);

$R^3$ is $C_{1-6}$ alkyl which may have one or more, preferably one to three halogen (e.g. methyl, trifluoromethyl);
acylamino such as $C_{1-6}$ alkanesulfonylamino (e.g. methanesulfonylamino);
carboxy($C_{1-6}$)alkoxy (e.g. carboxymethoxy);
$C_{1-6}$ alkoxycarbonyl($C_{1-6}$)alkoxy (e.g. ethoxycarbonylmethoxy);
halogen (e.g. fluoro, chloro);
$C_{1-6}$ alkoxy (e.g. methoxy); or
nitro;

$R^4$ is $C_{1-6}$ alkyl (e.g. methyl);

$R^5$ is mono or di or triphenyl($C_{1-6}$)alkyl, preferably phenyl($C_{1-6}$)alkyl (e.g. benzyl);

$R^7$ is hydrogen;
$C_{1-6}$ alkyl (e.g. methyl); or
halogen (e.g. chloro);

A is hydroxyproline (e.g. 4-hydroxyproline);
and

Y is bond;
$C_{1-6}$ alkylene (e.g. ethylene); or
$C_{2-6}$ alkenylene (e.g. vinylene).

The processes for preparing the object compound (I) are explained in detail in the following.

Process 1

The object compound (I) or a salt thereof can be prepared by reacting the compound (II) or its reactive derivative at the amino group or a salt thereof with the compound (III) or its reactive derivative at the carboxy group or a salt thereof.

Suitable reactive derivative at the amino group of the compound (II) may include Schiff's base type imino or its tautomeric enamine type isomer formed by the reaction of the compound (II) with a carbonyl compound such as aldehyde, or ketone; a silyl derivative formed by the reaction of the compound (II) with a silyl compound such as bis(trimethylsilyl)acetamide, mono(trimethylsilyl)acetamide, or bis(trimethylsilyl)urea; a derivative formed by reaction of the compound (II) with phosphorus trichloride or phosgene.

Suitable salts of the compound (II) and its reactive derivative can be referred to the ones as exemplified for the compound (I).

Suitable reactive derivative at the carboxy group of the compound (III) may include an acid halide, an acid anhydride, an activated amide, an activated ester. Suitable examples of the reactive derivatives may be an acid chloride; an acid azide; a mixed acid anhydride within acid such as substituted phosphoric acid [e.g. dialkylphosphoric acid, phenylphosphoric acid, diphenylphosphoric acid, dibenzylphosphoric acid, halogenated phosphoric acid], dialkylphosphorous acid, sulfurous acid, thiosulfuric acid, sulfuric acid, sulfonic acid [e.g. methanesulfonic acid], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid] or aromatic carboxylic acid [e.g. benzoic acid]; a symmetrical acid anhydride; an activated amide with imidazole, 4-substituted imidazole, dimethylpyrazole, triazole or tetrazole; or an activated ester [e.g. cyanomethyl ester, methoxymethyl ester, dimethyliminomethyl [$(CH_3)_2\overset{+}{N}=CH-$] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester], or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole]. These reactive derivatives can optionally be selected from them according to the kind of the compound (III) to be used.

Suitable salts of the compound (III) and its reactive derivative may be a base salt such as an alkali metal salt [e.g. sodium salt, potassium salt], an alkaline earth metal salt [e.g. calcium salt, magnesium salt], an ammonium salt, an organic base salt [e.g. trimethylamine salt, triethylamine salt, pyridine salt, picoline salt, dicyclohexylamine salt, N,N'-dibenzylethylenediamine salt], and an acid addition salt as exemplified for the compound (I).

The reaction is usually carried out in a conventional solvent such as water, alcohol [e.g. methanol, ethanol], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound (III) is used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide, N,N'-diisopropylcarbodiimide; N-ethyl-N'-(3-dimethylaminopropyl)carbodiimide; N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenyl phosphorylazide; thionyl chloride; oxalyl chloride; $C_1$-$C_6$ alkyl haloformate [e.g. ethyl chloroformate, isopropyl chloroformate]; triphenylphosphine; 2-ethyl-7-hydroxybenzisoxazolium salt; 2-ethyl-5-(m-sulfophenyl)isoxazolium hydroxide intramolecular salt; benzotriazol-1-yl-oxy-tris(dimethylamino)phosphoniumhexafluorophosphate; 1-(p-chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, phosphorus oxychloride.

The reaction may also be carried out in the presence of an inorganic or organic base such as an alkali metal bicarbonate, tri($C_1$-$C_6$)alkylamine, pyridine, N-($C_1$-$C_6$)alkylmorpholine, N,N-di($C_1$-$C_6$)alkylbenzylamine.

The reaction temperature is not critical, and the reaction is usually carried out under cooling to warming.

Process 2

The object compound (I-q) or a salt thereof can be prepared by subjecting the compound (I-p) or a salt thereof to elimination reaction of the carboxy protective group.

In the present elimination reaction, all conventional methods used in the elimination reaction of the carboxy protective group, for example, hydrolysis, reduction, elimination using Lewis acid are applicable. When the carboxy protective group is an ester, it can be eliminated by hydrolysis or elimination using Lewis acid. The hydrolysis is preferably carried out in the presence of a base or an acid.

Suitable base may include, for example, an inorganic base such as alkali metal hydroxide (e.g. sodium hydroxide, potassium hydroxide), alkaline earth metal hydroxide (e.g. magnesium hydroxide, calcium hydroxide), alkali metal carbonate (e.g. sodium carbonate, potassium carbonate), alkaline earth metal carbonate (e.g. magnesium carbonate, calcium carbonate), alkali metal bicarbonate (e.g. sodium bicarbonate, potassium bicarbonate), alkali metal acetate (e.g. sodium acetate, potassium acetate, etc.), alkaline earth metal phosphate (e.g. magnesium phosphate, calcium phosphate), alkali metal hydrogen phosphate (e.g. disodium hydrogen phosphate, dipotassium hydrogen phosphate), and an organic base such as trialkylamine (e.g. trimethylamine, triethylamine), picoline, N-methylpyrrolidine, N-methylmorpholine, 1,5-diazabicyclo[4.3.0]non-5-one, 1,4-diazabicyclo[2.2.2]octane, 1,5-diazabicyclo[5.4.0]undecene-5. The hydrolysis using a base is often carried out in water or a hydrophilic organic solvent or a mixed solvent thereof.

Suitable acid may include an organic acid (e.g. formic acid, acetic acid, propionic acid) and an inorganic acid (e.g. hydrochloric acid, hydrobromic acid, sulfuric acid).

The present hydrolysis is usually carried out in an organic solvent, water, or a mixed solvent thereof.

The reaction temperature is not critical, and it may suitably be selected in accordance with the kind of the carboxyprotective group and the elimination method.

The elimination using Lewis acid is carried out by reacting the compound (I-c) or a salt thereof with Lewis acid such as boron trihalide (e.g. boron trichloride, boron trifluoride), titanium tetrahalide (e.g. titanium tetrachloride, titanium tetrabromide), tin tetrahalide (e.g. tin tetrachloride, tin, tetrabromide), aluminum halide (e.g. aluminum chloride, aluminum bromide), trihaloacetic acid (e.g. trichloroacetic acid, trifluoroacetic acid). This elimination reaction is preferably carried out in the presence of cation trapping agents (e.g. anisole, phenol) and is usually carried out in a solvent such as nitroalkane (e.g. nitromethane, nitroethane), alkylene halide (e.g. methylene chloride, ethylene chloride), diethyl ether, carbon disulfide or any other solvent which does not adversely affect the reaction. These solvents may be used as a mixture thereof.

The reduction elimination can be applied preferably for elimination of the protective group such as halo($C_1$-$C_6$)alkyl (e.g. 2-iodoethyl, 2,2,2-trichloroethyl) ester, ar($C_1$-$C_6$)alkyl (e.g. benzyl) ester.

The reduction method applicable for the elimination reacting may include, for example, reduction by using a combination of a metal (e.g. zinc, zinc amalgam) or a salt of chromium compound (e.g. chromous chloride, chromous acetate) and an organic or an inorganic acid (e.g. acetic acid, propionic acid, hydrochloric acid); and conventional catalytic reduction in the presence of a conventional metallic catalyst (e.g. palladium carbon, Raney nickel).

The reaction temperature is not critical, and the reaction is usually carried out under cooling, at ambient temperature or under warming.

Process 3

The object compound (I-s) or a salt thereof can be prepared by subjecting the compound (I-r) or a salt thereof to reduction.

This reaction can be carried out in substantially the same manner as Process 2, and therefore the reaction mode and reaction conditions [e.g. bases, acids, reducing agents, catalysts, solvents, reaction temperature] of this reaction are to be referred to those as explained in Process 2.

The compounds obtained by the above processes can be isolated and purified by a conventional method such as pulverization, recrystallization, column chromatography, reprecipitation.

It is to be noted that the compound (I) and the other compounds may include one or more stereoisomers due to asymmetric carbon atoms, and all of such isomers and mixture thereof are included within the scope of this invention.

The object compounds (I) and pharmaceutically acceptable salt thereof have pharmacological activities such as tachykinin antagonism, especially substance P antagonism, neurokinin A antagonism or neurokinin B antagonism, and therefore are useful for treating or preventing tachykinin mediated diseases, for example, respiratory diseases such as asthma, bronchitis,rhinitis, cough, expectoration;
ophthalmic diseases such as conjunctivitis, vernal conjunctivitis;
cutaneous diseases such as contact dermatitis, atopic dermatitis, urticaria, and other eczematoid dermatitis; inflammatory diseases such as rheumatoid arthritis, osteoarthritis;
pains or aches (e.g. migraine, headache, toothache, cancerous pain, back pain).

Further, it is expected that the object compounds (I) of the present invention are useful for treating or preventing

ophthalmic diseases such as glaucoma, uveitis; gastrointestinal diseases such as ulcer, ulcerative colitis, irritable bowel syndrome, food allergy; inflammatory diseases such as nephritis; circulatory diseases such as hypertension, angina pectoris, cardiac failure, thrombosis; epilepsy; spastic paralysis; pollakiuria; dementia; Alzheimer's diseases; schizophrenia; Huntington's chorea; carcinoid syndrome; and useful for immunosuppresive agent.

For therapeutic purpose, the compounds (I) and pharmaceutically acceptable salts thereof of the present invention can be used in a form of pharmaceutical preparation containing one of said compounds, as an active ingredient, in admixture with a pharmaceutically acceptable carrier such as an organic or inorganic solid or liquid excipient suitable for oral, parenteral or external administration. The pharmaceutical preparations may be capsules, tablets, dragees, granules, solution, suspension, emulsion, or the like. If desired, there may be included in these preparation, auxiliary substances, stabilizing agents, wetting or emulsifying agents, buffers and other commonly used additives.

While the dosage of the compounds (I) will vary depending upon the age and condition of the patient, an average single dose of about 0.1 mg, 1 mg, 10 mg, 50 mg, 100 mg, 250 mg, 500 mg and 1000 mg of the compound (I) may be effective for treating asthma and the like. In general, amounts between 0.1 mg/body and about 1,000 mg/body may be administered per day.

In order to illustrate the usefulness of the object compound (I), the pharmacological test data of some representative compound of the compound (I) is shown in the following.

Test Compound :

(a)

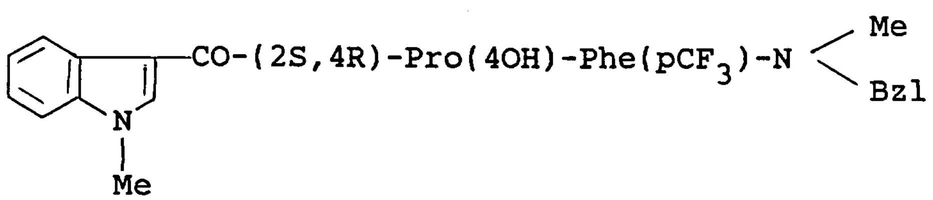

(1) $^{3}$H-Substance P receptor binding

Test Method :

(a) Crude lung membrane preparation

Male Hartley strain guinea pigs were sacrificed by decapitation. The trachea and lung were removed and homogenized in buffer (0.25 M sucrose, 50 mM Tris-HCl pH 7.5, 0.1 mM EDTA) by using Polytoron (Kinematica). The homogenate was centrifuged (1000 xg, 10 min) to remove tissue clumps and the supernatant was centrifuged (14000 xg 20 min) to yield pellets. The pellets were resuspended in buffer (5 mM Tris-HCl pH 7.5), homogenized with a teflon homogenizer and centrifuged (14000 xg, 20 min) to yield pellets which were referred to as crude membrane fractions. The obtained pellets were stored at -70°C until use.

(b) $^{3}$H-Substance P binding to preparation membrane

Frozen crude membrane fractions were thawed and resuspended in Medium 1 (50 mM Tris-HCl pH 7.5, 5 mM $MnCl_2$, 0.02% BSA, 2 μg/ml chymostatin, 4μg/ml leupeptin, 40 μg/ml bacitracin.) $^{3}$H-substance P (1 nM) was incubated with 100 μl of the membrane preparation in Medium 1 at 4°C for 30 minutes in a final volume of 500 μl. At the end of the incubation period, reaction mixture was quickly filtered over a Whatman GF/B glass filter (pretreated with 0.1% polyethylene imine for 3 hours prior to use) under aspiration. The filters were then washed four times with 5 ml of the buffer (50 mM Tris-HCl, pH 7.5). The radioactivity was counted in 5 ml of Aquazol-2 in Packerd scintillation counter (Packerd TRI -CARB 4530).

Test Result :

| Test Compound (0.1 μg/ml) | Inhibition (%) |
|---|---|
| (a) | 96 |

The following examples are given for purpose of illustrating the present invention in detail.

In these examples, there are employed the following abbreviations in addition to the abbreviations adopted by the IUPAC-IUB.

| | |
|---|---|
| Ac | : acetyl |
| Aib | : 2-aminoisobutyric acid |
| Azt | : azetidine-2-carboxylic acid |
| Boc | : t-butoxycarbonyl |
| BSA | : bistrimethylsilylacetamide |
| $Bu^t$ | : t-butyl |
| Bz | : benzoyl |
| Bzl | : benzyl |
| DMAP | : dimethylaminopyridine |
| DMF | : dimethylformamide |
| DMSO | : dimethylsulfoxide |
| Et | : ethyl |
| HOBT | : N-hydroxybenzotriazole |
| IPE | : isopropyl ether |
| Me | : methyl |
| Ms | : mesyl |
| NMM | : N-methylmorpholine |
| HCl/DOX | : hydrogen chloride in 1,4-dioxane |
| $Pr^i$ | : isopropyl |
| Py(2) | : 2-pyridyl |
| Su | : succinimido |
| TEA | : triethylamine |
| TFA | : trifluoroacetic acid |
| THF | : tetrahydrofuran |
| Tpr | : thioproline |
| Ts | : tosyl |
| WSC | : 1-ethyl-3-(3'-dimethylaminopropyl)carbodiimide |
| Z | : benzyloxycarbonyl |

The Starting Compounds used and the Object Compounds obtained in the following examples are given in The Table as below, in which the formulae of the former compounds are in the upper and the formulae of the latter compounds are in the lower, respectively.

Table

| Preparation No. | Formula |
|---|---|
| 1 | H-Phe(p-$CF_3$)-OH |
| | Boc-Phe(p-$CH_3$)-OH |
| 2 - (1) | H-Phe(p-F)-OH |
| | Boc-Phe(p-F)-OH |
| 2 - (2) | H-Phe(o-F)-OH |
| | Boc-Phe(o-F)-OH |
| 2 - (3) | H-Phe(m-F)-OH |
| | Boc-Phe(m-F)-OH |
| 2 - (4) | H-Phe(m-$CF_3$)-OH |
| | Boc-Phe(m-$CF_3$)-OH |
| 3 | Boc-Phe(p-$CF_3$)-OH |
| | Boc-Phe(p-$CF_3$)-N 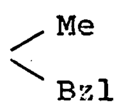 |

| Preparation No. | Formula |
|---|---|
| 4 - (1) | Boc-Phe(p-$NO_2$)-OH |
| | Boc-Phe(p-$NO_2$)-N< Me, Bzl |
| 4 - (2) | Boc-Phe(o-F)-OH |
| | Boc-Phe(o-F)-N< Me, Bzl |
| 4 - (3) | Boc-Phe(p-F)-OH |
| | Boc-Phe(p-F)-N< Me, Bzl |
| 4 - (4) | Boc-Phe(m-F)-OH |
| | Boc-Phe(m-F)-N< Me, Bzl |
| 4 - (5) | Boc-Phe(m-$CF_3$)-OH |
| | Boc-Phe(m-$CF_3$)-N< Me, Bzl |
| 4 - (6) | Boc-Phe(p-OH)-OH |
| | Boc-Phe(p-OH)-N< Me, Bzl |
| 5 | Boc-Phe(p-OH)-N< Me, Bzl |
| | Boc-Phe(p-OMe)-N< Me, Bzl |

| Preparation No. | Formula |
|---|---|
| 6 | 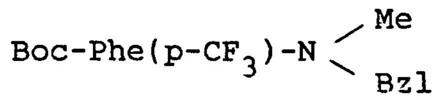 |
| | 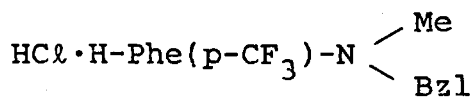 |
| 7 - (1) | 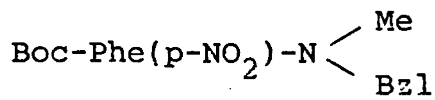 |
| | 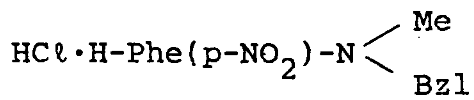 |
| 7 - (2) | 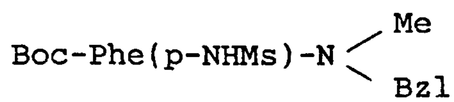 |
| | 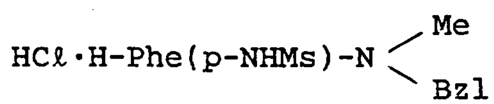 |
| 7 - (3) | 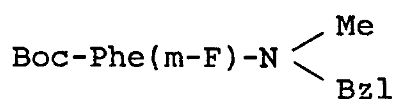 |
| | 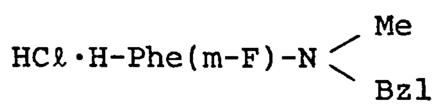 |
| 7 - (4) | 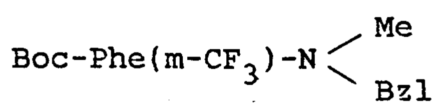 |
| | 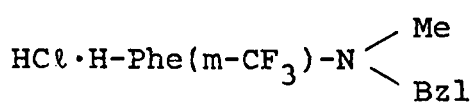 |
| 7 - (5) | 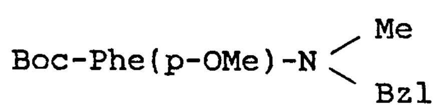 |
| | 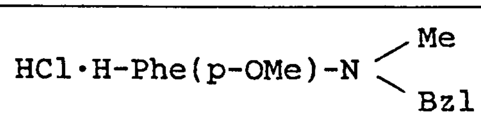 |

| Preparation No. | Formula |
|---|---|
| 8 | HCℓ·H-Phe(p-$CF_3$)-N$<^{Me}_{Bzl}$ |
| | Boc-(2S,4R)-Pro(4OH)-Phe(p-$CF_3$)-N$<^{Me}_{Bzl}$ |
| 9 - 1 | HCℓ·H-Phe(p-$NO_2$)-N$<^{Me}_{Bzl}$ |
| | Boc-(2S,4R)-Pro(4OH)-Phe(p-$NO_2$)-N$<^{Me}_{Bzl}$ |
| 9 - 2 | HCℓ·H-Phe(p-NHMs)-N$<^{Me}_{Bzl}$ |
| | Boc-(2S,4R)-Pro(4OH)-Phe(p-NHMs)-N$<^{Me}_{Bzl}$ |
| 9 - 3 | HCℓ·H-Phe(m-F)-N$<^{Me}_{Bzl}$ |
| | Boc-(2S,4R)-Pro(4OH)-Phe(m-F)-N$<^{Me}_{Bzl}$ |
| 9 - 4 | HCℓ·H-Phe(m-$CF_3$)-N$<^{Me}_{Bzl}$ |
| | Boc-(2S,4R)-Pro(4OH)-Phe(m-$CF_3$)-N$<^{Me}_{Bzl}$ |

| Preparation No. | Formula |
|---|---|
| 9 - 5 | 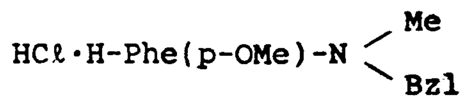 |
| | 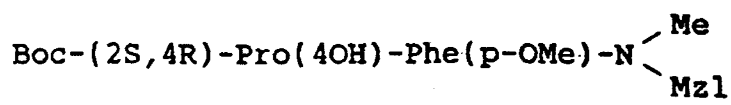 |
| 10 | 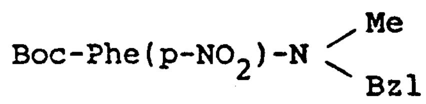 |
| | 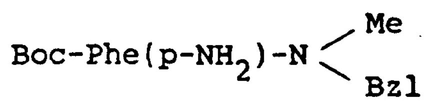 |
| 11 | 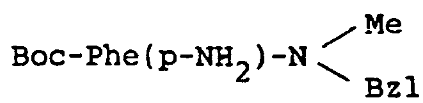 |
| | 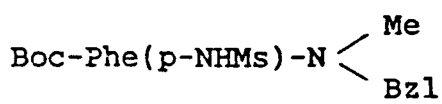 |
| 12 - (1) | 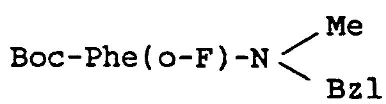 |
| | 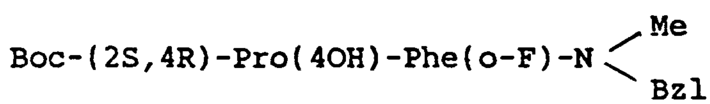 |
| 12 - (2) | 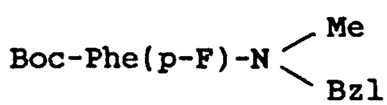 |
| | 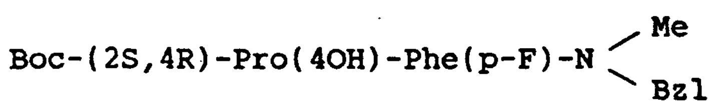 |
| 12 - (3) | 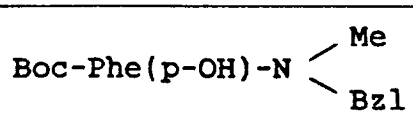 |
| | 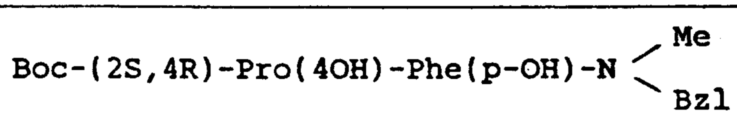 |

| Preparation NO | Formula |
|---|---|
| 13 | Boc-(2S,4R)-Pro(4OH)-Phe(p-OH)-N$<^{Me}_{Bzl}$ |
| | Boc-(2S,4R)-Pro(4OH)-Phe(p-$OCH_2CO_2Et$)-N$<^{Me}_{Bzl}$ |
| 14 | Boc-(2S,4R)-Pro(4OH)-Phe(p-$CF_3$)-N$<^{Me}_{Bzl}$ |
| | HCℓ·H-(2S,4R)-Pro(4OH)-Phe(p-$CF_3$)-N$<^{Me}_{Bzl}$ |
| 15 - (1) | Boc-(2S,4R)-Pro(4OH)-Phe(p-$NO_2$)-N$<^{Me}_{Bzl}$ |
| | HCℓ·H-(2S,4R)-Pro(4OH)-Phe(p-$NO_2$)-N$<^{Me}_{Bzl}$ |
| 15 - (2) | Boc-(2S,4R)-Pro-(4OH)-Phe(p-NHMs)-N$<^{Me}_{Bzl}$ |
| | HCℓ·H-(2S,4R)-Pro(4OH)-Phe(p-NHMs)-N$<^{Me}_{Bzl}$ |
| 15 - (3) | Boc-(2S,4R)-Pro(4OH)-Phe(o-F)-N$<^{Me}_{Bzl}$ |
| | HCℓ·H-(2S,4R)-Pro(4OH)-Phe(o-F)-N$<^{Me}_{Bzl}$ |
| 15 - (4) | Boc-(2S,4R)-Pro(4OH)-Phe(p-F)-N$<^{Me}_{Bzl}$ |
| | HCℓ·H-(2S,4R)-Pro(4OH)-Phe(p-F)-N$<^{Me}_{Bzl}$ |

| Preparation No. | Formula |
|---|---|
| 15 - (5) | Me<br>Boc-(2S,4R)-Pro(4OH)-Phe(m-F)-N<br>Bzl |
| | Me<br>HCℓ·H-(2S,4R)-Pro(4OH)-Phe(m-F)-N<br>Bzl |
| 15 - (6) | Me<br>Boc-(2S,4R)-Pro(4OH)-Phe(p-$OCH_2CO_2$Et)-N<br>Bzl |
| | Me<br>HCℓ·H-(2S,4R)-Pro(4OH)-Phe(p-$OCH_2CO_2$Et)-N<br>Bzl |
| 15 - (7) | Me<br>Boc-(2S,4R)-Pro(4OH)-Phe(m-$CF_3$)-N<br>Bzl |
| | Me<br>HCℓ·H-(2S,4R)-Pro(4OH)-Phe(m-$CF_3$)-N<br>Bzl |
| 15 - (8) | Me<br>Boc-(2S,4R)-Pro(4OH)-Phe(p-OMe)-N<br>Bzl |
| | Me<br>HCℓ·H(2S,4R)-Pro(4OH)-Phe(p-OMe)-N<br>Bzl |
| 16 | H-Phe(3,4-diMe)-OH |
| | Boc-Phe(3,4-diMe)-OH |
| 17 | Boc-Phe(3,4-diMe)-OH |
| | Me<br>Boc-Phe(3,4-diMe)-N<br>Bzl |

| Preparation NO | Formula |
|---|---|
| 18 | 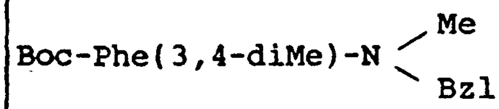 |
| | 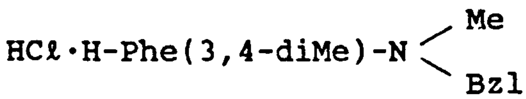 |
| 19 | 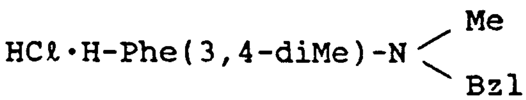 |
| | 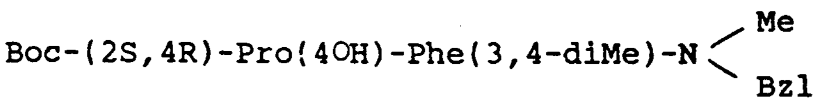 |
| 20 | 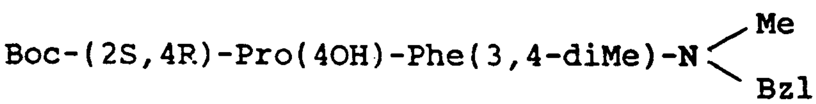 |
| | 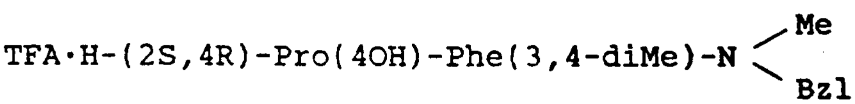 |

| Example No. | Formula |
|---|---|
| 1 | 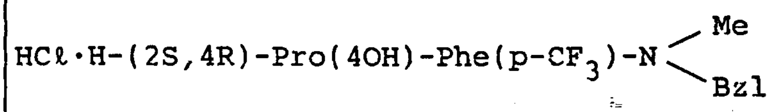 |
| | 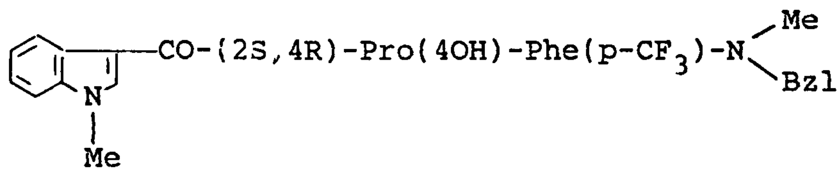 |
| 2-(1) | 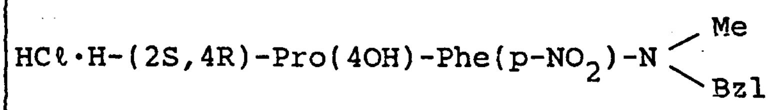 |
| | 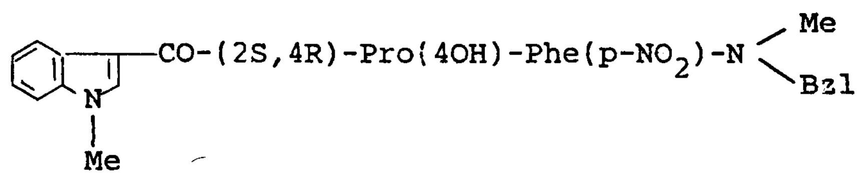 |
| 2-(2) | 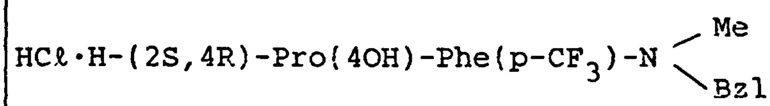 |
| | 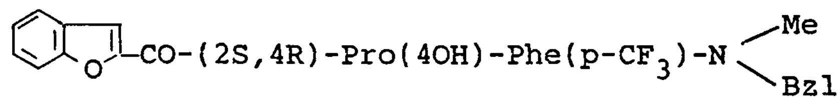 |
| 2-(3) | 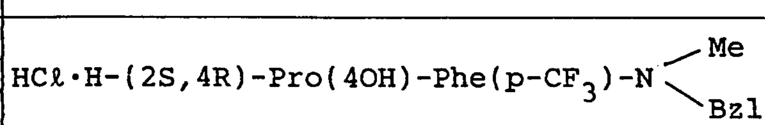 |
| | 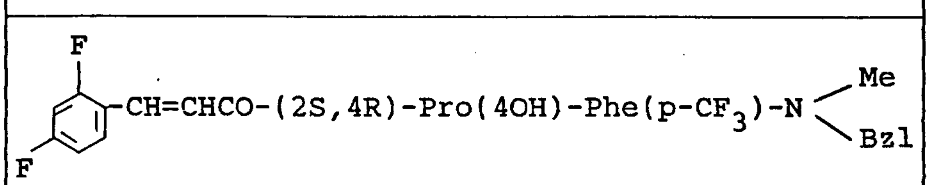 |
| 2-(4) | 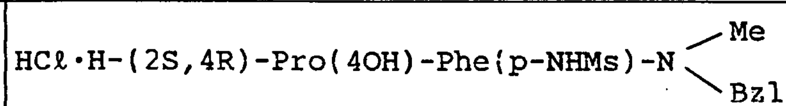 |
| | 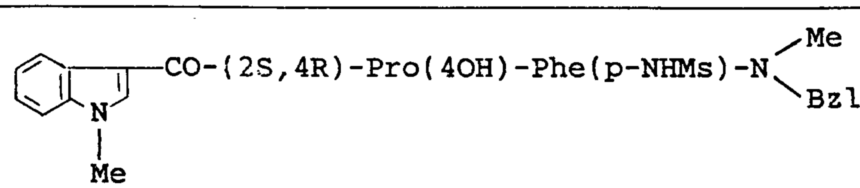 |

| Example No. | Formula |
|---|---|
| 2-(5) | 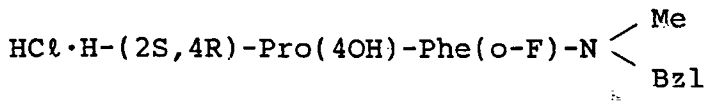 |
| | 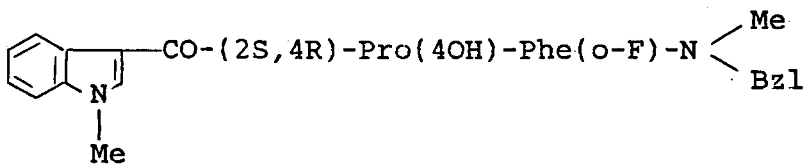 |
| 2-(6) | 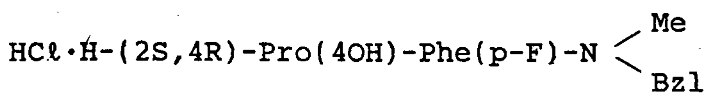 |
| | 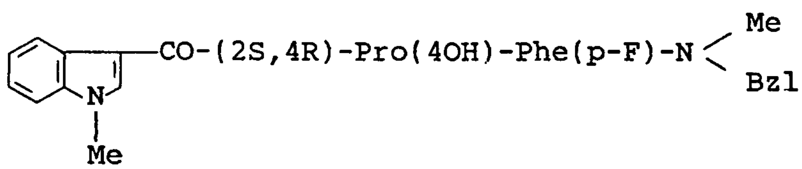 |
| 2-(7) | 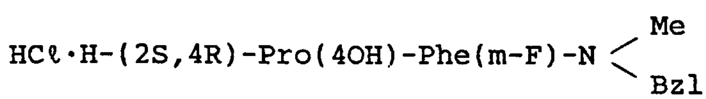 |
| | 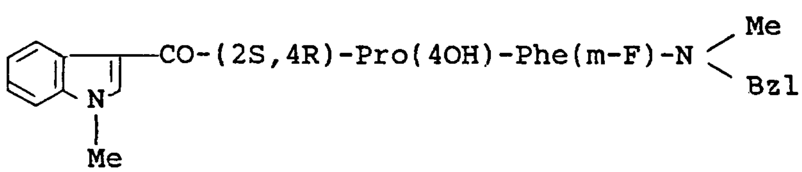 |
| 2-(8) | 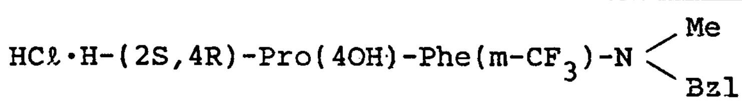 |
| | 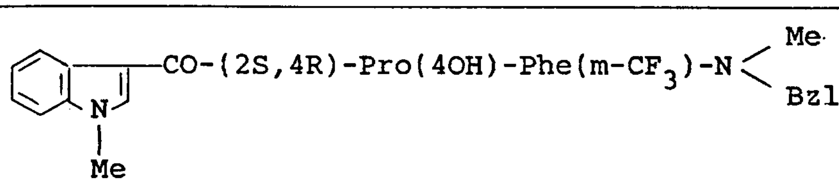 |
| 2-(9) | 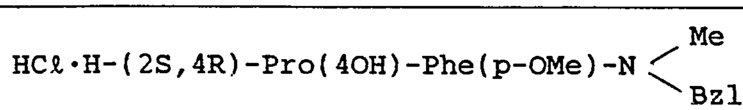 |
| | 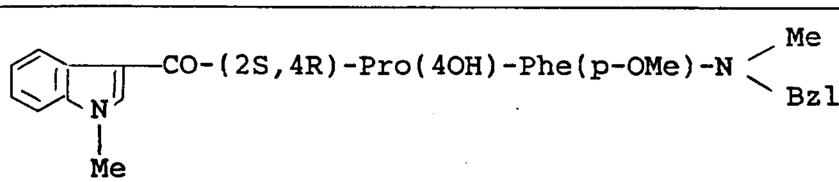 |

| Example No. | Formula |
|---|---|
| 3 | 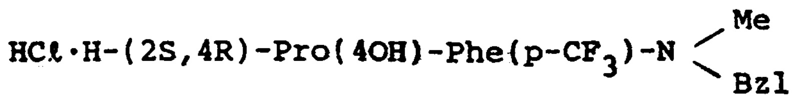 |
| | 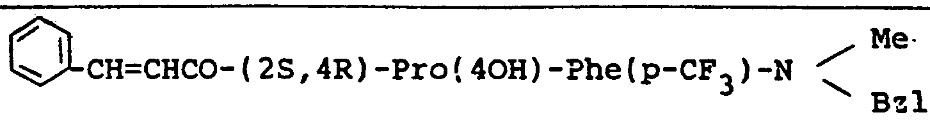 |
| 4 | 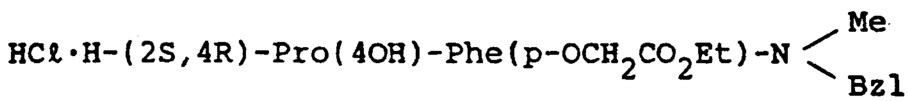 |
| | 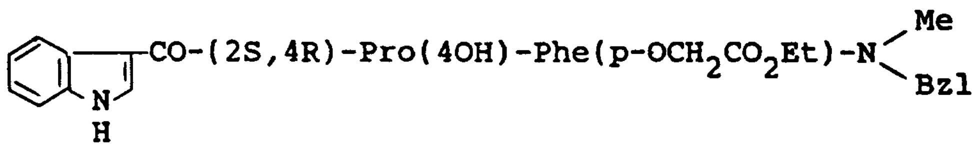 |
| 5 | 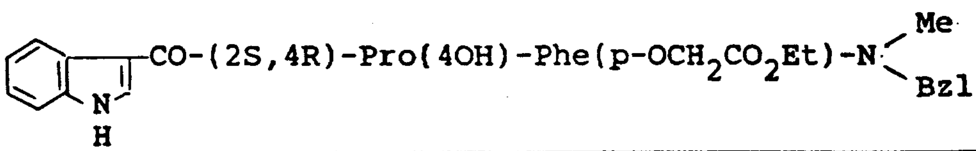 |
| | 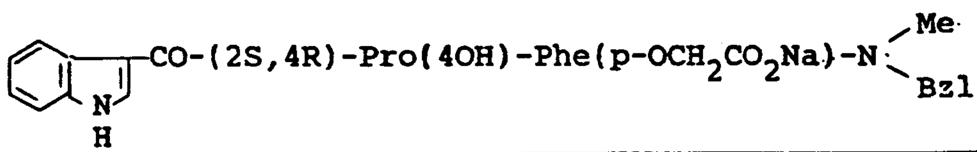 |
| 6 | 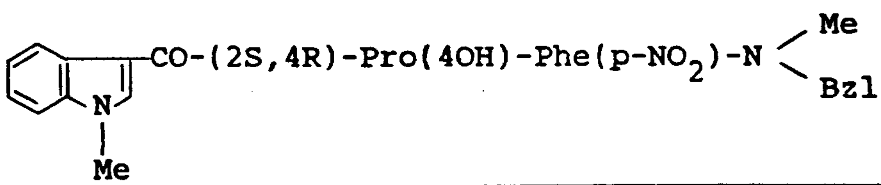 |
| | 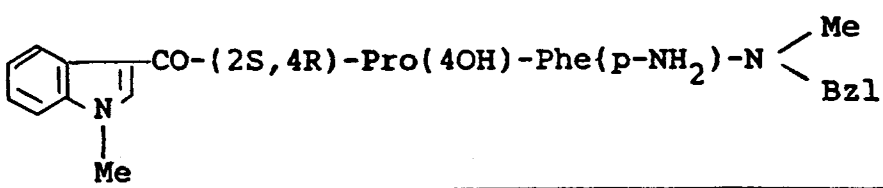 |

| Example No. | Formula |
|---|---|
| 7 | 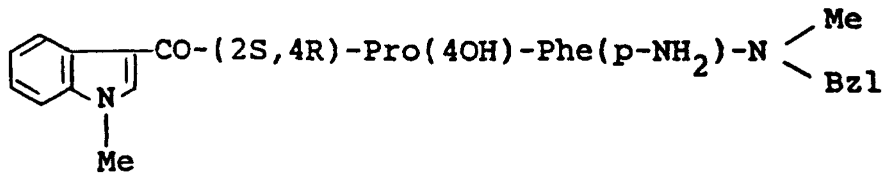 |
| | 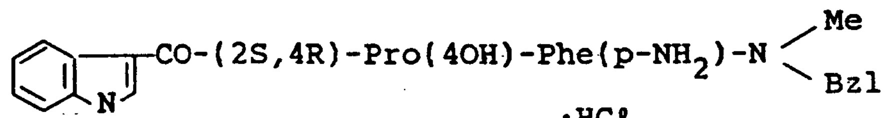 •HCl |
| 8 | 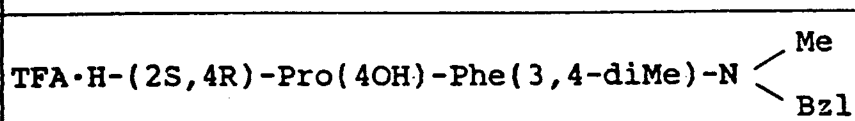 |
| | 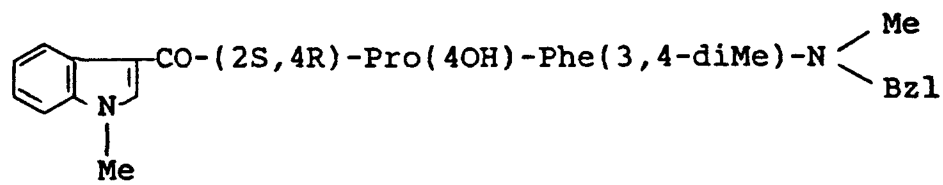 |

Preparation 1

To a suspended mixture of Starting Compound (5.0 g) in a mixed solvent of water (35 ml) and acetone (35 ml) was added TEA (4.47 ml) under ice-cooling. To the solution was added a solution of di-tert-butyldicarbonate (5.6 g) in acetone (20 ml), and the solution was stirred at the same temperature for 1 hour and at room temperature for additional 1 hour, during which period, di-tert-butyldicarbonate (1.0 g) was added. After removal of the acetone, water was added and the aqueous solution was washed once with ether. The aqueous layer was then acidified to pH 2 with an addition of 6N hydrochloric acid and was extracted with ethyl acetate. The extract was washed with an aqueous sodium chloride solution and was dried over magnesium sulfate. After evaporation, the residue was crystallized from a mixed solvent of diisopropyl ether and n-hexane, and was collected by filtration and dried to give Object Compound (6.58 g).

mp : 116°C

IR (Nujol) : 3370, 1715, 1690, 1620, 1520 $cm^{-1}$

NMR (DMSO-$d_6$, δ) : 1.30 (9H, s), 2.90 (1H) and 3.14 (1H)(ABX.$J_{AB}$=13.7Hz, $J_{AC}$=4.4Hz, $J_{BC}$=10.7Hz), 4.17 (1H, m), 7.18 (1H, d, J=8.6Hz), 7.48 (1H, d, J=8Hz), 7.65 (1H, d, J=8Hz)

Preparation 2

The object compounds were obtained according to a similar manner to that of Preparation 1.

(1)

IR (Neat) : 3320, 2980, 1715, 1510 $cm^{-1}$

NMR (DMSO-$d_6$, δ) : 1.26 (s) and 1.31 (s)(9H), 2.7-2.9 (1H, m), 2.9-3.1 (1H, m), 3.9-4.2 (1H, m), 7.0-7.2 (3H, m), 7.2-7.4 (2H, m), 12.63 (1H, broad)

(2)

IR (Nujol) : 3380, 1720, 1685, 1520 $cm^{-1}$
NMR ($DMSO-d_6$, δ) : 1.24 (s) and 1.30 (s)(9H), 2.7-2.9 (1H, m), 3.0-3.2 (1H, m), 4.0-4.2 (1H, m), 7.0-7.4 (5H, m), 12.67 (1H, broad)

(3)

mp : 73-74°C
IR (Nujol) : 3370, 1715, 1690, 1620, 1580, 1520 $cm^{-1}$
NMR ($DMSO-d_6$, δ) : 1.32 (9H, s), 2.92 (1H, d of ABq, J=14Hz, 10Hz), 3.06 (1H, d of ABq, J=14Hz, 5Hz), 4.0-4.2 (1H, m), 7.0-7.2 (4H, m), 7.3-7.4 (1H, m), 12.51 (1H, br s)

(4)

IR (Nujol) : 3380, 2660, 2620, 1760, 1640, 1630, 1405 $cm^{-1}$
NMR ($DMSO-d_6$, δ) : 1.22 (s) and 1.29 (s)(9H), 2.91 (1H, dd, J=11Hz, 14Hz), 3.15 (1H, dd, J=4Hz, 14Hz), 4.16 (1H, m), 7.19 (1H, d, J=9Hz), 7.4-7.7 (4H, m), 12.72 (1H, broad)

Preparation 3

To an ice-cooled solution of Starting Compound (2 g), N-methylbenzylamine (0.77 ml), and HOBT (0.81 g) in methylene chloride (50 ml), was added WSC • HCl (1.15 g). The solution was stirred at the same temperature for an hour and at room temperature overnight. After evaporation, the reaction mixture was extracted with ethyl acetate, and the organic layer was washed successively with water, an aqueous sodium hydrogencarbonate solution, 0.5N hydrochloric acid, water and an aqueous sodium chloride solution, and was dried over magnesium sulfate. Evaporation of this solution gave Object Compound (2.88 g).

mp : 86-88°C
IR (Nujol) : 3370, 1685, 1640, 1520 $cm^{-1}$
NMR ($DSMO-d_6$, δ) : 1.12 (s) and 1.31 (s)(9H), 2.77 and 2.91 (3H, s), 4.4-4.8 (3H, m), 7.1-7.4 (6H, m), 7.4-7.7 (4H, m), 2.8-3.1 (2H, m)

Preparation 4

The following compounds were obtained according to a similar manner to that of Preparation 3.

(1)

mp : 110-111°CC
IR (Nujol) : 3390, 1685, 1645, 1600, 1515, 1450, 1345, 1260, 1165 $cm^{-1}$
NMR ($DMSO-d_6$, δ) : 1.12 (s) and 1.31 (s)(9H), 2.7-3.1 (5H, m), 4.3-4.8 (3H, m), 7.1-7.5 and 7.5-7.7 and 8.1-8.2 (10H, m)

(2)

IR (Neat) : 3320, 2990, 1710, 1640, 1490 $cm^{-1}$
NMR ($DMSO-d_6$, δ) : 1.21 (s) and 1.32 (s)(9H), 2.7-3.1 (2H, m), 2.73 (s) and 2.90 (s)(3H), 4.3-4.8 (3H, m), 7.0-7.4 (10H, m)

(3)

IR (Nujol) : 3370, 1685, 1635, 1520 $cm^{-1}$
NMR ($DMSO-d_6$, δ): 1.24 (s) and 1.34 (s)(9H), 2.7-3.0 (2H, m), 2.75 (s) and 2.88 (s)(3H), 4.3-4.7 (3H, m), 7.0-7.4 (10H, m)

(4)

mp : 94-96°C

IR (Nujol) : 3380, 1690, 1645, 1635, 1525, 1450, 1250, 1170 $cm^{-1}$
NMR (DMSO-$d_6$, δ) : 1.23 (s) and 1.33 (9H, s), 2.7-2.9 (5H, m), 4.3-4.7 (3H, m), 6.9-7.4 (10H, m)

(5)

IR (Nujol) : 3370, 1685, 1640, 1520 $cm^{-1}$
NMR (DMSO-$d_6$, δ) : 1.19 (s) and 1.30 (s)(9H), 2.77 (s), and 2.91 (s)(3H), 2.8-3.1 (2H, m), 4.3-4.8 (3H, m), 7.0-7.7 (10H, m)

(6)

IR (Nujol) : 3320, 1685, 1640, 1530, 1515, 1455, 1415, 1265, 1170 $cm^{-1}$
NMR (DMSO-$d_6$, δ) : 1.2-1.4 (9H, m), 2.6-2.9 (5H, m), 4.3-4.7 (3H, m), 6.5-6.7 and 5.8-6.9 and 6.9-7.4 (10H, m), 9.20 (1H, s)

Preparation 5

To a solution of Starting Compound (1.54 g) in THF (30 ml) were added sodium hydride (60% in oil, 176 mg) and methyl iodide (0.5 ml). The mixture was stirred at 60°C for an hour. After concentration, the product was extracted with ethyl acetate, and the organic layer was washed successively with water and an aqueous sodium chloride solution and was dried over magnesium sulfate. Evaporation of this solution gave Object Compound (1.78 g) as an oil.

IR ($CHCl_3$) : 3320, 1705, 1640, 1510, 1450, 1365, 1250, 1170 $cm^{-1}$
NMR (DMSO-$d_6$, δ) : 1.2-1.4 (9H, m), 2.7-2.9 (5H, m), 3.70 (s) and 3.72 (s)(3H), 4.3-4.6 (3H, m), 6.7-6.9 (2H, m), 7.0-7.4 (8H, m)

Preparation 6

To an ice-cooled solution of Starting Compound (2.05 g) in methylene chloride (20 ml) was added 4N-HCL/DOX (19.0 ml). The solution was stirred at the same temperature for 5 minutes. Then the cooling bath was removed and the solution was stirred at room temperature for an hour. After evaporation, the residue was triturated with diisopropyl ether, collected by filtration, and dried over sodium hydroxide in vacuo to give Object Compound (1.68 g).

mp : 159°C
IR (Nujol) : 1650, 1605, 1580, 1495, 1335 $cm^{-1}$
NMR (DMSO-$d_6$, δ) : 2.73 (3H, s), 3.1-3.3 (2H, m), 4.1-4.8 (3H, m), 7.1-7.2 (2H, m), 7.2-7.4 (3H, m), 7.37 (2H, d, J=8Hz), 7.62 (d, J=8Hz) and 7.69 (d, J=8Hz)(2H), 8.51 (2H, broad s)

Preparation 7

The object compounds were obtained according to a similar manner to that of Preparation 6.

(1)

IR ($CHCl_3$) : 1655, 1605, 1525, 1350, 1215 $cm^{-1}$
NMR (DMSO-$d_6$, δ) : 2.72 (s) and 2.74 (s)(3H), 3.1-3.3 (2H, m), 4.1-4.8 (3H, m), 7.1-7.3 (5H, m), 7.52 (2H, d, J=8Hz), 8.1-8.2 (2H, m), 8.61 (3H, br s)

(2)

IR ($CHCl_3$) : 1655, 1640, 1610, 1510, 1490, 1450, 1400, 1330, 1215 $cm^{-1}$
NMR (DMSO-$d_6$, δ) : 2.64 (s) and 2.70 (s)(3H), 2.9-3.2 (5H, m), 4.3-4.7 (3H, m), 7.1-7.2 and 7.3-7.4 (9H, m), 8.47 (3H, br s), 9.8-9.9 (1H, m)

(3)

IR ($CHCl_3$) : 1655, 1590, 1490, 1450, 1255, 1215 $cm^{-1}$
NMR (DMSO-$d_6$, δ) : 2.71 (s) and 2.73 (3H, s), 3.0-3.3 (2H, m), 4.1-4.8 (3H, m), 7.0-7.2 and 7.2-7.4 (9H, m), 8.49 (3H, br s)

(4)

IR (Nujol) : 3500, 3450, 1650, 1610 $cm^{-1}$

NMR (DMSO-$d_6$, δ) : 2.70 (s) and 2.75 (s)(3H), 3.1-3.3 (2H, m), 4.09 and 4.67 (ABq, J=16Hz) and 4.46 (s)(2H), 4.75 (1H, t, J=7Hz), 7.0-7.2 (2H, m), 7.2-7.4 (3H, m), 7.5-7.6 (2H, m), 7.6-7.7 (2H, m), 8.49 (3H, broad s)

(5)

IR ($CHCl_3$) : 1660, 1650, 1615, 1515, 1250, 1220 $cm^{-1}$

NMR (DMSO-$d_6$, δ) : 2.59 (s) and 2.69 (s)(3H), 2.9-3.2 (2H, m), 3.72 (s) and 3.76 (s)(3H), 4.3-4.5 (3H, m), 6.75-6.95 (2H, m), 7.1-7.2 and 7.3-7.4 (7H, m), 8.50 (3H, br s)

Preparation 8

To an ice-cooled solution of Starting Compound (1.65 g), Boc-(2S,4R)-Pro(4OH)-OH (1.02 g) and HOBT (0.60 g) in a mixed solvent of methylene chloride (45 ml) and dimethylformamide (10 ml) was added WSC (0.80 ml). The solution was stirred at the same temperature for an hour and at room temperature overnight. After evaporation, the reaction mixture was extracted with ethyl acetate and the organic layer was washed successively with an aqueous sodium hydrogencarbonate solution, water, 0.5N hydrochloric acid, water and an aqueous sodium chloride solution, and was dried over magnesium sulfate and evaporated in vacuo to give Object Compound (2.35 g) as an oil.

IR ($CHCl_3$) : 1700, 1685, 1665, 1645, 1635, 1555, 1540 $cm^{-1}$

NMR (DMSO-$d_6$, δ) : 1.1-1.3 (m) and 1.65 (s)(9H), 1.5-1.8 (1H, m), 1.8-2.1 (1H, m), 2.9 (3H, s), 2.7-3.1 (2H, m), 3.1-3.4 (1H, m), 3.4-3.5 (1H, m), 4.1-4.3 (2H, m), 4.3-4.5 (2H, m), 4.7-5.1 (2H, m), 7.0-7.7 (9H, m), 8.39 (1H, d, J=8Hz)

Preparation 9

The object compounds were obtained according to a similar manner to that of Preparation 8.

(1)

IR ($CHCl_3$) : 1690-1630, 1605, 1520, 1455, 1345, 1160 $cm^{-1}$

NMR (DMSO-$d_6$, δ) : 1.12 (s), 1.22 (s) and 1.37 (s)(9H), 1.5-1.8 (1H, m), 1.8-2.1 (1H, m), 2.7-3.1 (5H, m), 3.1-3.5 (2H, m), 4.0-4.3 (2H, m), 4.3-4.6 (2H, m), 4.7-5.2 (2H, m), 7.0-7.5 (m), 7.5-7.7 (m), 8.0-8.2 (m) and 8.32 (s)(9H), 8.42 (1H, d, J=8Hz)

(2)

IR ($CHCl_3$) : 1690, 1680, 1630, 1510, 1400, 1330, 1155 $cm^{-1}$

NMR (DMSO-$d_6$, δ) : 1.26 (s) and 1.39 (s)(9H), 1.5-1.8 (1H, m), 1.8-2.1 (1H, m), 2.7-3.1 (8H, m), 3.2-3.3 (1H, m), 3.3-3.5 (1H, m), 4.1-4.3 (2H, m), 4.3-5.1 (4H, m), 6.9-7.1 and 7.1-7.4 (9H, m), 8.2-8.4 (1H, m), 9.63 (1H, br s)

(3)

IR ($CHCl_3$) : 3400-3200, 1690, 1680, 1640, 1590, 1550, 1450, 1410, 1365, 1250 $cm^{-1}$

NMR (DMSO-$d_6$, δ) : 1.22 (s) and 1.38 (9H, s), 1.5-1.8 (1H, m), 1.8-2.1 (1H, m), 2.7-3.1 (5H, m), 3.1-3.3 (1H, m), 3.3-3.5 (1H, m), 4.1-4.3 (2H, m), 4.4-4.6 (2H, m), 4.8-5.1 (2H, m), 6.8-7.4 (9H, m), 8.3-8.4 (1H, m)

(4)

IR (Neat) : 3450-3300, 1690-1650, 1640 $cm^{-1}$

NMR (DMSO-$d_6$, δ) : 1.19 (s), 1.21 (s) and 1.38 (s)(9H), 1.6-1.8 (1H, m), 1.8-2.1 (1H, m), 2.7-3.5 (7H, m), 4.0-4.2 (2H, m), 4.3-4.7 (2H, m), 4.7-5.1 (2H, m), 7.0-7.2 (2H, m), 7.2-7.7 (7H, m), 8.3-8.5 (1H, m)

(5)

IR ($CHCl_3$) : 3450-3300, 1695-1630, 1510, 1405, 1250, 1160 $cm^{-1}$
NMR (DMSO-$d_6$, δ) : 1.25 (s) and 1.39 (s)(9H), 1.5-1.8 (1H, m), 1.8-2.1 (1H, m), 2.7-3.0 (5H, m), 3.2-3.3 (1H, m), 3.4-3.5 (1H, m), 3.71 (3H, s), 4.1-4.3 (2H, m), 4.3-4.6 and 4.7-5.0 (4H, m), 6.7-6.9 (2H, m), 6.9-7.1 and 7.1-7.3 (7H, m), 8.2-8.4 (1H, m)

Preparation 10

To a solution of Starting Compound (2.5 g) in methanol (70 ml) was added 10% palladium on charcoal (0.5 g), and the mixture was hydrogenated under atmospheric pressure at room temperature for 1.5 hours. Filtration and concentration of the mixture gave Object Compound (2.27 g) as an amorphous solid.

IR ($CHCl_3$) : 3370, 1705, 1640, 1520, 1365, 1290, 1250, 1170 $cm^{-1}$
NMR (DMSO-$d_6$, δ) : 1.1-1.4 (9H, m), 2.5-2.9 (5H, m), 4.3-4.6 (3H, m), 4.91 (2H, s), 6.4-6.5 (2H, m), 6.74 (1H, d, J=8Hz), 6.89 (1H, d, J=8Hz), 7.0-7.2 and 7.2-7.4 (6H, m)

Preparation 11

To an ice-cooled solution of Starting Compound (1.75 g) in pyridine (35 ml) was added methanesulfonyl chloride (0.39 ml). The solution was stirred for an hour at the same temperature. After concentration, the product was extracted with ethyl acetate and the organic layer was washed successively with water, diluted aqueous sodium hydrogen carbonate solution, water, 0.5N hydrochloric acid and sodium chloride solution, and was dried over magnesium sulfate. Evaporation of the extract gave Object Compound (2.14 g) as an amorphous solid.

IR ($CHCl_3$) : 1710-1690, 1635, 1510, 1495, 1400, 1335, 1250 $cm^{-1}$
NMR (DMSO-$d_6$, δ) : 1.26 (s) and 1.34 (s)(9H), 2.7-2.9 (5H, m), 2.92 (3H, s), 4.4-4.7 (3H, m), 7.0-7.4 (10H, m), 9.62 (1H, s)

Preparation 12

The object compounds were obtained according to similar manner to that of Preparation 6 and Preparation 8, successively.

(1)

IR (Neat) : 3400, 3320, 1680 (broad), 1640, 1490 $cm^{-1}$
NMR (DMSO-$d_6$, δ) : 1.24 (s) and 1.39 (s)(9H), 1.5-1.8 (1H, m), 1.8-2.1 (1H, m), 2.7-3.5 (7H, m), 4.1-4.3 (2H, m), 4.4-4.8 (2H, m), 4.9-5.2 (2H, m), 7.0-7.4 (9H, m), 8.2-8.5 (1H, m)

(2)

IR (Neat) : 3450-3300 (broad), 1690-1660 (broad), 1640, 1510 $cm^{-1}$
NMR (DMSO-$d_6$, δ) : 1.23 (s) and 1.39 (s)(9H), 1.5-1.8 (1H, m), 1.8-2.1 (1H, m), 2.7-3.1 (5H, m), 3.1-3.5 (2H, m), 4.0-4.3 (2H, m), 4.3-5.1 (4H, m), 7.0-7.4 (9H, m), 8.35 (1H, d, J=8Hz)

(3)

IR (Nujol) : 3280, 1665, 1630, 1515 $cm^{-1}$
NMR (DMSO-$d_6$, δ) : 1.28 (s) and 1.39 (s)(3H), 1.60-1.90 (1H, m), 1.90-2.10 (1H, m), 2.60-3.00 (2H, m), 2.75 (s) and 2.85 (s)(3H), 3.20-3.30 (1H, m), 3.35-3.50 (1H, m), 4.10-4.70 (4H, m) 4.70-5.05 (2H, m), 6.60 (d, J=8Hz) and 6.64 (d, J=8Hz)(2H), 6.86 (d, J=8Hz) and 7.03 (d, J=8Hz)(2H), 6.90-7.10 (2H, m), 7.20-7.35 (3H, m), 8.20-8.40 (1H, m), 9.19 (s) and 9.23 (s)(1H)

Preparation 13

To a solution of Starting Compound (5.0 g) in methylene chloride (120 ml) were added cetyltrimethylammonium chloride (300 mg) and powdered sodium hydroxide (0.80 g). Then, ethyl bromoacetate (2.02 g) was added to the mixture and the mixture was stirred for two hours at room temperature. After addition of acetic acid (1 ml) and concentration, ethyl acetate and water were added to the residue. The separated aqueous layer was extracted with ethyl acetate again and the organic layers were combined and washed successively with an aqueous sodium hydrogencarbonate solution, 0.5N hydrochloric acid, and an aqueous sodium chloride solution, and dried over magnesium sulfate. Concentration of this solution gave crude product (3.83 g), which was purified on a silica gel column eluting with a mixed solvent of methylene chloride and methanol (97:3 to 94:6) to give Object Compound (3.13 g) as an amorphous solid.

IR ($CH_2Cl_2$) : 3600, 3400, 1750, 1680, 1640 $cm^{-1}$
NMR ($CDCl_3$, δ) : 1.30 (3H, t), 1.45 (9H, s), 1.8-2.2 (2H, m), 2.71 and 2.91 (3H, s), 2.9-3.1 (3H, m), 3.3-3.55 (2H, m), 4.26 (2H, g), 4.0-4.5 (4H, m), 4.55-4.6 (3H, m), 5.1-5.2 (1H, m), 6.7-6.8 (2H, m), 7.0-7.2 (4H, m), 7.25-7.3 (3H, m)

Preparation 14

To an ice-cooled solution of Starting Compound (1.1 g) in methylene chloride (11 ml) was added 4N-HCl/DOX (8.1 ml). The solution was stirred at the same temperature for 5 minutes and at room temperature for 50 minutes. After evaporation, the residue was triturated with diisopropyl ether, collected by filtration and dried to give Object Compound (0.88 g).

IR (Nujol) : 3250, 1670, 1645, 1580, 1555 $cm^{-1}$
NMR (DMSO-$d_6$, δ) : 1.6-1.9 (1H, m), 2.2-2.4 (1H, m), 2.79 (s) and 2.89 (s)(3H), 2.9-3.4 (4H, m), 4.2-4.6 (4H, m), 4.9-5.2 (1H, m), 5.5-5.6 (1H, m), 7.0-7.7 (10H, m), 9.23 (1H, d, J=8Hz)

Preparation 15

The object compounds were obtained according to a similar manner to that of Preparation 14.

(1)

mp : 188-189°C
IR (Nujol) : 3500, 3290, 2700, 1665, 1645, 1605, 1565, 1515, 1345 $cm^{-1}$
NMR (DMSO-$d_6$, δ) : 1.6-1.9 (1H, m), 2.1-2.4 (1H, m), 2.7-3.4 (7H, m), 4.1-4.7 (4H, m), 4.9-5.2 (1H, m), 5.5-5.6 (1H, m), 7.0-7.6 (7H, m), 8.0-8.2 (2H, m), 8.51 (br s) and 10.0 (br s)(1H), 9.25 (1H, d, J=8Hz)

(2)

IR ($CHCl_3$) : 3320, 3020, 2850, 2700, 1660, 1640, 1630, 1530, 1510, 1390, 1215 $cm^{-1}$
NMR (DMSO-$d_6$, δ) : 1.7-1.9 (1H, m), 2.2-2.4 (1H, m), 2.7-3.2 (8H, m), 3.2-3.4 (2H, m), 4.2-4.7 and 4.9-5.1 (6H, m), 7.0-7.2 and 7.2-7.4 (9H, m), 8.67 (1H, br s), 9.16 (1H, d, J=8Hz), 9.7-9.8 (1H, m), 9.98 (1H, br s)

(3)

NMR (DMSO-$d_6$, δ) : 1.6-1.9 (1H, m), 2.2-2.4 (1H, m), 2.75 (s) and 2.86 (s)(3H), 2.8-3.4 (4H, m), 4.2-4.7 (4H, m), 5.0-5.2 (1H, m), 5.57 (1H, s), 7.0-7.4 (9H, m), 8.62 (1H, broad), 9.23 (1H, d, J=8Hz), 10.00 (1H, broad)

(4)

NMR (DMSO-$d_6$, δ) : 1.6-1.9 (1H, m), 2.2-2.4 (1H, m), 2.77 (s) and 2.86 (s)(3H), 2.8-3.2 (3H, m), 3.2-3.5 (1H, m), 4.2-4.7 (4H, m), 4.8-5.1 (1H, m), 5.56 (1H, s), 7.0-7.4 (9H, m), 8.58 (1H, broad), 9.17 (1H, d, J=8Hz), 9.98 (1H, broad)

(5)

mp : 216-217°C
IR (Nujol) : 3410, 3200, 3070, 1675, 1640, 1615, 1560, 1450, 1265 $cm^{-1}$
NMR (DMSO-$d_6$, δ) : 1.7-1.9 (1H, m), 2.2-2.4 (1H, m), 2.7-3.2 and 3.2-3.4 (7H, m), 4.2-4.7 (4H, m), 4.9-5.1

(1H, m), 5.58 (1H, s), 6.9-7.4 (9H, m), 8.58 (1H, br s), 9.1-9.3 (1H, m), 10.03 (1H, br s)

(6)

mp : 137-141°C
IR (Nujol) : 3300, 1750, 1670, 1640, 1560, 1510 $cm^{-1}$
NMR (DMSO-$d_6$, δ) : 1.21 (3H, t, J=7Hz), 1.7-1.9 (1H, m), 2.2-2.4 (1H, m), 2.74 and 2.83 (3H, s), 2.8-3.1 (2H, m), 3.2-3.4 (2H, m), 4.16 (2H, q, J=7Hz), 4.3-4.6 (4H, m), 4.74 (2H, s), 4.8-5.0 (1H, m), 5.59 (1H, m), 6.8-6.9 (2H, m), 7.05-7.4 (7H, m), 9.16 (1H, d, J=7.7Hz)

(7)

NMR (DMSO-$d_6$, δ) : 1.7-1.9 (1H, m), 2.2-2.4 (1H, m), 2.78 (s) and 2.89 (s)(3H), 2.9-3.4 (4H, m), 4.2-4.7 (4H, m), 4.9-5.1 (1H, m), 5.58 (1H, d, J=3Hz), 7.0-7.2 (2H, m), 7.2-7.4 (3H, m), 7.4-7.7 (4H, m), 9.20 (1H, d, J=8Hz)

(8)

IR ($CHCl_3$) : 1675, 1635, 1550, 1510, 1450, 1300, 1250, 1180 $cm^{-1}$
NMR (DMSO-$d_6$, δ) : 1.7-1.9 (1H, m), 2.2-2.4 (1H, m), 2.7-3.1 and 3.2-3.4 (7H, m), 3.72 (3H, s), 4.2-4.6 (4H, m), 4.8-5.0 (1H, m), 5.57 (1H, br s), 6.8-6.9 (2H, m), 7.0-7.4 (7H, m), 8.59 (1H, br s), 9.13 (1H, d, J=8Hz), 10.04 (1H, br s)

Preparation 16

The object compound was obtained according to a similar manner to that of Preparation 1.

IR ($CHCl_3$) : 3470, 1725, 1715, 1500 $cm^{-1}$
NMR (DMSO-$d_6$, δ) : 1.33 (9H, s), 2.17 (6H, s), 2.65-3.0 (2H, m), 3.95-4.1 (1H, m), 6.9-7.05 (4H, m), 12.46 (1H, br s)

Preparation 17

The object compound was obtained according to a similar manner to that of Preparation 3.

IR (Neat) : 3450, 3320, 1710, 1640, 1365 $cm^{-1}$
NMR (DMSO-$d_6$, δ) : 1.2-1.4 (9H, m), 2.1-2.2 (6H, m), 2.65-2.95 (5H, m), 4.3-4.7 (3H, m), 6.75-7.4 (9H, m)

Preparation 18

The object compound was obtained according to a similar manner to that of Preparation 6.

mp : 96-104°C
IR (Nujol) : 3440, 1650, 1610, 1490, 1450, 1280 $cm^{-1}$
NMR (DMSO-$d_6$, δ) : 2.1-2.25 (6H, m), 2.62 (s) and 2.69 (3H, s), 2.85-3.2 (2H, m), 4.0-4.1 and 4.35-4.65 (3H, m), 6.9-7.4 (8H, m), 8.49 (3H, br s)

Preparation 19

The object compound was obtained according to a similar manner to that of Preparation 8.

IR ($CHCl_3$) : 3450-3300, 1690-1650, 1645-1625, 1450, 1155 $cm^{-1}$
NMR (DMSO-$d_6$, δ) : 1.2-1.45 (9H, m), 1.6-1.85 (1H, m), 1.9-2.2 (7H, m), 2.7-3.0 (5H, m), 3.15-3.5 (2H, m), 4.1-5.1 (6H, m), 6.7-7.35 (8H, m), 8.25-8.35 (1H, m)

Preparation 20

The object compound was obtained according to a similar manner to that of Preparation 14 excepting for using TFA in place of 4N-HCl/DOX.

IR ($CHCl_3$) : 3450-3200, 1680, 1640, 1565, 1455 $cm^{-1}$

NMR (DMSO-$d_6$, δ) : 1.7-1.9 (1H, m), 2.1-2.4 (7H, m), 2.65-3.15 (5H, m), 3.25-3.5 (2H, m), 4.2-5.1 and 5.55-5.65 (6H, m), 6.8-7.4 (8H, m), 9.1-9.3 (1H, m), 8.65 (1H, br s), 10.0 (1H, br s)

Example 1

To an ice-cooled solution of 1-methylindole-3-carboxylic acid (0.23 g), Starting Compound (0.63 g), methylene chloride (12 ml) and HOBT (0.18 g) was added WSC (0.24 ml). The solution was stirred at the same temperature for an hour and at room temperature overnight. After evaporation, the reaction mixture was extracted with ethyl acetate and the organic layer was washed successively with an aqueous sodium hydrogencarbonate solution, water, 0.5N hydrochloric acid, water, and an aqueous sodium chloride solution, and dried over magnesium sulfate. After evaporation, the residue was purified on a silica gel column (100 g) eluting with a mixed solvent of chloroform and methanol (50:1). The fractions containing the desired compound were collected and evaporated. The residue was then crystallized from ethyl acetate, filtered collected by filtration and dried to give Object Compound (0.50 g).

mp : 183-184°C

IR (Nujol) : 3270, 1680, 1655, 1580, 1570, 1530 $cm^{-1}$

NMR (DMSO-$d_6$, δ) : 1.7-1.9 (1H, m), 1.9-2.1 (1H, m), 2.72 (s) and 2.85 (s)(3H), 2.9-3.3 (2H, m), 3.66 (1H, d, J=5Hz), 3.8-4.1 (1H, m), 3.85 (3H, s), 4.30 (s) and 4.43 (s) and 4.6-4.8 (m)(4H) 4.9-5.2 (2H, m), 7.0-7.7 (12H, m), 7.88 (1H, s), 8.07 (1H, d, J=8Hz), 8.4-8.7 (1H, m)

Example 2

The object compounds were obtained according to a similar manner to that of Example 1.

(1)

mp : 183-184°C

IR (Nujol) : 3280, 1690, 1650, 1585, 1545, 1520, 1450, 1350 $cm^{-1}$

NMR (DMSO-$d_6$, δ) : 1.7-1.9 (1H, m), 1.9-2.1 (1H, m), 2.6-3.3 (5H, m), 3.5-3.7 (1H, m), 3.7-4.0 (1H, m), 3.85 (3H, s), 4.2-4.7 (4H, m), 4.9-5.1 (2H, m), 7.0-7.6 (10H, m), 7.8-8.1 (4H, m), 8.4-8.6 (1H, m)

(2)

mp : 124-126°C

IR (Nujol) : 3420, 3300, 1670, 1640, 1615, 1545, 1325, 1160, 1126, 1110, 1066 $cm^{-1}$

NMR (DMSO-$d_6$, δ) : 1.6-2.4 (2H, m), 2.6-3.3 (5H, m), 3.6-4.1 (2H, m), 4.2-4.8 (4H, m), 5.0-5.2 (2H, m), 6.8-7.9 (14H, m), 8.6-8.9 (1H, m)

(3)

mp : 98-100°C

IR (Nujol) : 3300, 1650, 1630, 1614, 1588, 1530, 1504, 1330 $cm^{-1}$

NMR (DMSO-$d_6$, δ) : 1.6-2.2 (2H, m), 2.5-3.2 (5H, m), 3.5-3.9 (2H, m), 4.2-4.8 (4H, m), 4.9-5.2 (2H, m), 6.7-6.8, 7.0-7.6, 7.7-7.8, 8.0-8.2, 8.5-8.6 and 8.9-9.0 (14H, m)

(4)

IR ($CHCl_3$) : 1640, 1630, 1530, 1510, 1465, 1435, 1330, 1220, 1150 $cm^{-1}$

NMR (DMSO-$d_6$, δ) : 1.7-1.9 (1H, m), 1.9-2.1 (1H, m), 2.6-3.1 (5H, m), 2.89 (3H, s), 3.6-3.7 (1H, m), 3.8-4.0 (1H, m), 3.86 (3H, s), 4.2-4.6, 4.6-4.8 and 4.8-5.1 (6H, m), 7.0-7.5 (12H, m), 7.8-8.0 (1H, m), 8.06 (1H, d, J=8Hz), 8.3-8.5 (1H, br s), 9.64 (1H, br s)

(5)

IR (Nujol) : 3420, 3290, 3100, 1655, 1640, 1600, 1570, 1535 $cm^{-1}$

NMR (DMSO-$d_6$, δ) : 1.7-1.9 (1H, m), 1.9-2.1 (1H, m), 2.67 (s) and 2.83 (s)(3H), 2.8-3.2 (2H, m), 3.6-3.7 (1H, m), 3.7-4.0 (4H, m), 4.2-5.0 (4H, m), 4.9-5.2 (1H, m), 5.01 (1H, d, J=3Hz), 6.9-7.4 (11H, m), 7.50 (1H, d, J=8Hz), 7.89 (1H, br s), 8.05 (1H, d, J=8Hz), 8.3-8.5 (1H, m)

(6)

| | |
|---|---|
| IR (Nujol) : | 3430, 3270, 3100, 1655, 1635, 1605, 1570, 1535, 1510 $cm^{-1}$ |
| NMR (DMSO-$d_6$, δ) : | 1.7-1.9 (1H, m), 1.9-2.1 (1H, m), 2.70 (s) and 2.81 (s)(3H), 2.8-3.1 (2H, m), 3.6-3.7 (1H, m), 3.7-4.0 (4H, m), 4.2-4.6 (3H, m), 4.6-4.8 (1H, m), 4.8-5.1 (1H, m), 5.02 (1H, d, J=3Hz), 6.9-7.4 (11H, m), 7.49 (1H, d, J=8Hz), 7.90 (1H, br s), 8.06 (1H, d, J=8Hz), 8.3-8.5 (1H, m) |

(7)

| | |
|---|---|
| mp : | 187-188°C |
| IR (Nujol) : | 3350, 3270, 1685, 1650, 1590, 1545, 1450, 1250 $cm^{-1}$ |
| NMR (DMSO-$d_6$, δ) : | 1.7-1.9 (1H, m), 1.9-2.1 (1H, m), 2.7-3.1 (5H, m), 3.6-3.8 and 3.8-4.0 (2H, m), 3.86 (3H, s), 4.2-4.5 (3H, m), 4.6-4.8 (1H, m), 4.9-5.1 (2H, m), 6.9-7.3 (11H, m), 7.4-7.5 (1H, m), 7.8-8.0 (1H, m), 8.0-8.1 (1H, m), 8.3-8.5 (1H, m) |

(8)

| | |
|---|---|
| IR (Nujol) : | 3360, 1685, 1655, 1580, 1565, 1530 $cm^{-1}$ |
| NMR (DMSO-$d_6$, δ) : | 1.6-1.8 (1H, m), 1.8-2.1 (1H, m), 2.71 (s) and 2.85 (s)(3H), 2.8-3.3 (2H, m), 3.6-3.7 (1H, m), 3.7-4.0 (1H, m), 3.86 (3H, s), 4.2-4.7 (4H, m), 4.8-5.1 (1H, m), 5.00 (1H, d, J=3Hz), 6.9-7.3 (7H, m), 7.3-7.7 (5H, m), 7.89 (1H, broad s), 8.05 (1H, d, J=8Hz), 8.3-8.6 (1H, m) |

(9)

| | |
|---|---|
| mp : | 172-173°C |
| IR (Nujol) : | 3430, 3280, 1655, 1630-1600, 1510, 1450, 1235 $cm^{-1}$ |
| NMR (DMSO-$d_6$, δ) : | 1.7-1.9 (1H, m), 1.9-2.1 (1H, m), 2.7-3.0 (5H, m), 3.6-3.8 (1H, m), 3.69 (3H, s), 3.8-4.0 (1H, m), 3.85 (3H, s), 4.3-4.6, 4.6-4.8 and 4.8-5.1 (6H, m), 6.7-6.8, 6.9-7.3 and 7.5-7.6 (12H, m), 7.8-8.0 and 8.0-8.1 (2H, m), 8.3-8.5 (1H, m) |

## Example 3

To a suspended mixture of Starting Compound (1.0 g) in methylene chloride (25 ml) was added pyridine (0.65 g) and cinnamoyl chloride (0.343 g) under ice-cooling. Tetrahydrofuran (5 ml) was added to the mixture and the resulting solution was stirred at the same temperature for one and half an hour and at room temperature for two hours. After concentration, the product was extracted with ethyl acetate and the organic layer was washed successively with diluted sodium hydrogen carbonate solution, water, 0.5N hydrochloric acid, and sodium chloride solution, and was dried with magnesium sulfate. After concentration, the residue was crystallized with a mixed solvent of ethyl acetate, diisopropyl ether, and ether, and was filtered and washed with diisopropyl ether to give Object Compound (1.13 g).

| | |
|---|---|
| mp : | 91-94°C |
| IR (Nujol) : | 3350 (sh), 3290, 1665, 1645, 1610, 1585, 1530 $cm^{-1}$ |

## Example 4

To an ice-cooled solution of Starting Compound (1.61 g) and BSA (1.89 g) in methylene chloride (35 ml) was added indole-3-carbonyl chloride (0.70 g). The solution was stirred at this temperature for two hours. To the evaporated residue was added a mixture of THF (20 ml) and water (5 ml). The mixture was stirred at room temperature for 20 minutes. The solution was washed with water, diluted sodium hydrogencarbonate solution, 0.5N hydrochloric acid and sodium chloride solution and dried over magnesium sulfate. After concentration, the residue was applied to a silica gel (42 g) column and eluted firstly with chloroform and secondly with chloroform-methanol (100:1:5 to 100:2 gradient elution) to give Object Compound (1.89 g).

IR (Nujol) : 3250, 1750, 1630, 1510 $cm^{-1}$

NMR (DMSO-$d_6$, δ) : 1.20 (3H, t, J=7Hz), 1.7-2.1 (2H, m), 2.65-3.0 (5H, m), 3.6-3.7 and 3.8-4.0 (2H, m), 4.15 (2H, q, J=7Hz), 4.2-4.5 (3H, m), 4.69 (2H, s), 4.7 (1H, m), 4.8-5.0 (2H, m), 6.7-6.8 (2H, m), 7.0-7.5 (10H, m), 7.85 (1H, br s), 8.03 (1H, d, J=7.3Hz), 8.4 (1H, m), 11.64 (1H, br s)

Example 5

To an ice-cooled solution of Starting Compound (727 mg) in ethanol (15 ml) was added a solution of 1N sodium hydroxide (2.57 ml). The solution was stirred at room temperature for 3 hours. After evaporation of alcohol, water was added and the solution was lyophilized to give Object Compound (660 mg) as a powder.

IR (Nujol) : 3200, 1610, 1515 $cm^{-1}$
NMR (DMSO-$d_6$, δ) : 1.8-2.1 (2H, m), 2.7-3.0 (including singlets at 2.72 and 2.85) (5H, m), 3.55-3.65 and 3.7-3.9 (2H, m), 4.13 (2H, s), 4.2 (1H, m), 4.4-4.6 (including singlet at 4.44)(2H), 4.68 (1H, m), 4.89 (1H, m), 5.24 (1H, s), 6.6-7.4 (11H, m), 7.49 (1H, d, J=7Hz), 7.8 (1H, br s), 8.02 (1H, d, J=7Hz), 8.31 (1H, m)

Example 6

Starting Compound (0.6 g) was dissolved in methanol (40 ml) and hydrogenated over 10% palladium on charcoal (0.06 g) under atmospheric pressure for an hour. The catalyst was filtered off and the filtrate was concentrated to give Object Compound (0.61 g) as an amorphous solid.

IR ($CHCl_3$) : 3360, 1630, 1530, 1515, 1470, 1435, 1370, 1250 $cm^{-1}$
NMR (DMSO-$d_6$, δ) : 1.7-1.9 (1H, m), 1.9-2.1 (1H, m), 2.6-2.9 (5H, m), 3.6-3.7 (1H, m), 3.85 (3H, s), 3.8-4.0 (1H, m), 4.3-4.5 (3H, m), 4.6-5.1 (5H, m), 6.4-6.5, 6.7-7.1, 7.1-7.3 and 7.4-7.5 (12H, m), 7.8-8.0 (1H, m), 8.0-8.1 (1H, m), 8.3-8.4 (1H, m)

Example 7

Starting Compound (0.6 g) was dissolved in tetrahydrofuran (6 ml). To the solution was added 4N-HCl/DOX (0.28 ml) under ice-cooling. The solution was stirred for ten minutes and concentrated. The residue was triturated with ether and filtered and dried (40°C, 6 hours) to give Object Compound (0.58 g).

mp : >173°C (dec.)
IR (Nujol) : 3500-3100, 1630, 1530, 1510, 1250 $cm^{-1}$
NMR (DMSO-$d_6$, δ) : 1.7-1.9 (1H, m), 1.9-2.1 (1H, m), 2.7-3.2 (5H, m), 3.6-3.7 (1H, m), 3.8-4.2 (4H, m), 7.0-7.6 (12H, m), 7.91 (1H, s), 8.06 (1H, d, J=7Hz), 8.3-8.6 (1H, m), 4.2-5.1 (6H, m), 10.3 (3H, br s)

Example 8

The object compound was obtained according to a similar manner to that of Example 1.

mp : >105°C (dec.)
IR (Nujol) : 3430-3280, 1645-1630, 1605, 1530, 1415 $cm^{-1}$
NMR (DMSO-$d_6$, δ) : 1.75-1.95 (1H, m), 2.0-2.2 (7H, m), 2.70-2.78 (3H, s), 2.8-3.05 (2H, m), 3.6-4.0 (2H, m), 3.85 (3H, s), 4.25-5.05 (6H, m), 6.75-7.5 (11H, m), 7.8-8.1 (2H, m), 8.3-8.5 (1H, m)

## Claims

**Claims for the following Contracting States : AT, BE, CH, DE, DK, FR, GB, IT, LI, LU, NL, SE**

1. A compound of the formula :

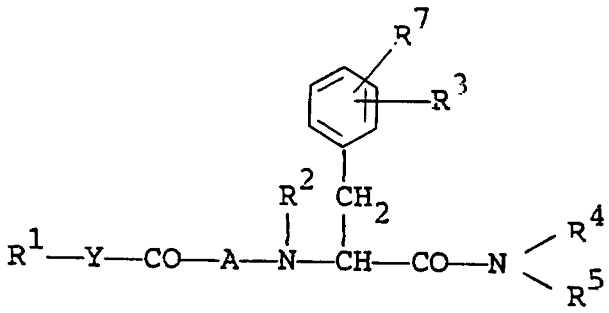

wherein

| | |
|---|---|
| $R^1$ | is $C_6$-$C_{10}$ aryl which may have one to three substituent(s) selected from halogen and $C_{1-6}$ alkoxy;<br>benzofuryl;<br>pyridyl;<br>or a group of the formula : |

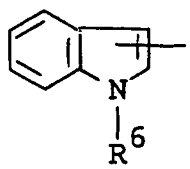

| | |
|---|---|
| | wherein $R^6$ is hydrogen; or<br>$C_{1-6}$ alkyl, |
| $R^2$ | is hydrogen or $C_{1-6}$ alkyl, |
| $R^3$ | is $C_{1-6}$ alkyl which may have one to three halogen;<br>amino;<br>acylamino;<br>carboxy($C_{1-6}$)alkoxy;<br>$C_{1-6}$ alkoxycarbonyl($C_{1-6}$)alkoxy;<br>halogen;<br>$C_{1-6}$ alkoxy; or nitro, |
| $R^4$ | is $C_{1-6}$ alkyl, |
| $R^5$ | is mono or di or triphenyl($C_{1-6}$)alkyl, |
| $R^7$ | is hydrogen; or<br>$C_{1-6}$ alkyl; |
| A | is hydroxyproline, and |
| Y | is bond, $C_{1-6}$ alkylene or $C_{2-6}$ alkenylene, |

and a pharmaceutically acceptable salt thereof.

**2.** A compound of claim 1, wherein

| | |
|---|---|
| $R^1$ | is phenyl which may have one to two halogen or one to two $C_{1-6}$ alkoxy, benzofuryl, pyridyl, indolyl or 1-$C_{1-6}$ alkylindolyl, |
| $R^3$ | is $C_{1-6}$ alkyl, trihalo($C_{1-6}$)alkyl, amino, $C_{1-6}$ alkanesulfonylamino, carboxy($C_{1-6}$)alkoxy, $C_{1-6}$ alkoxycarbonyl($C_{1-6}$)alkoxy, halogen, $C_{1-6}$ alkoxy or nitro, |
| $R^5$ | is phenyl($C_{1-6}$)alkyl, and |
| A | is hydroxyproline. |

**3.** A compound of claim 2, wherein

| | |
|---|---|
| $R^1$ | is phenyl, difluorophenyl, dimethoxyphenyl, benzofuryl, pyridyl, indolyl, or 1-methylindolyl, |

| | |
|---|---|
| $R^2$ | is hydrogen or methyl, |
| $R^3$ | is methyl, trifluoromethyl, amino, methanesulfonylamino, carboxymethoxy, ethoxycarbonylmethoxy, fluoro, chloro, methoxy or nitro, |
| $R^4$ | is methyl, |
| $R^5$ | is benzyl, |
| $R^7$ | is hydrogen or methyl, and |
| Y | is bond, ethylene or vinylene. |

**4.** A compound of claim 3, which is selected from the group consisting of :

(1) 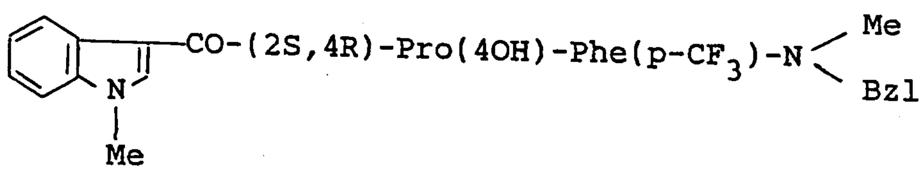

(2) 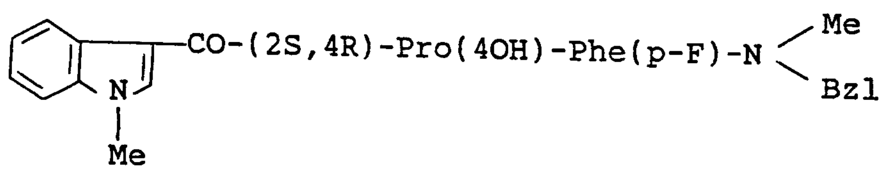

(3) 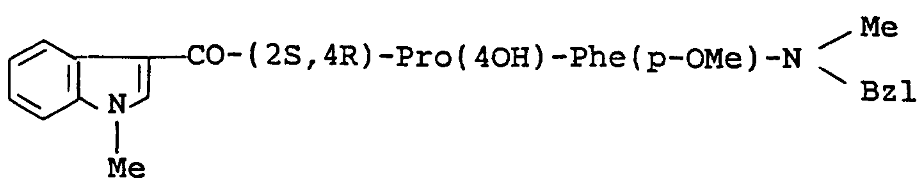

**5.** A process for preparing a compound of Claim 1 or a pharmaceutically acceptable salt thereof, which comprises

(1) reacting a compound of the formula :

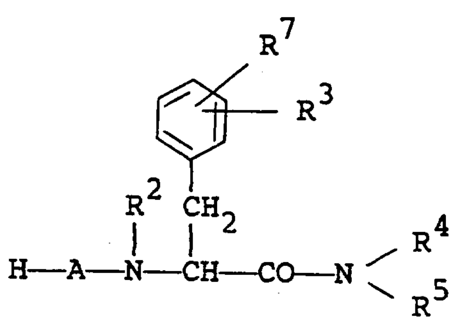

wherein $R^2$, $R^3$, $R^4$, $R^5$, $R^7$ and A are each as defined above,
or its reactive derivative at the amino group or a salt thereof, with a compound of the formula :

$R^1$ - Y - COOH

wherein $R^1$ and Y are each as defined above,
or its reactive derivative at the carboxy group or a salt thereof, to give a compound of the formula:

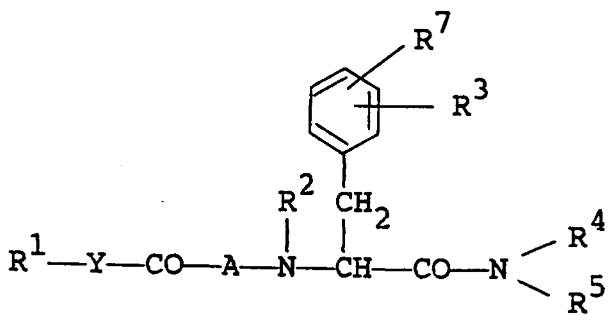

wherein $R^1$, $R^2$, $R^3$, $R^4$, $R^5$, $R^7$, A and Y are each as defined above,
or a salt thereof, or

(2) subjecting a compound of the formula :

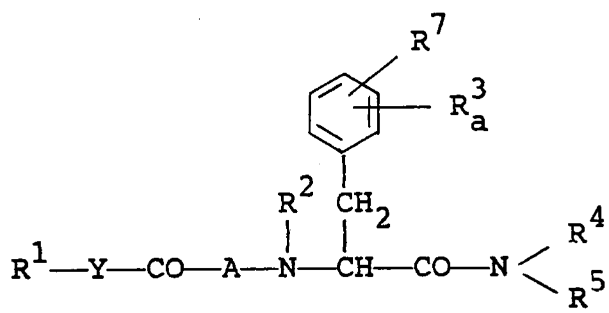

wherein $R^1$, $R^2$, $R^4$, $R^5$, A, $R^7$ and Y are each as defined above, and
$R^3_a$ is $C_{1-6}$ alkoxycarbonyl($C_{1-6}$)alkoxy,
or a salt thereof, to elimination reaction of the carboxy protective group, to give a compound of the formula :

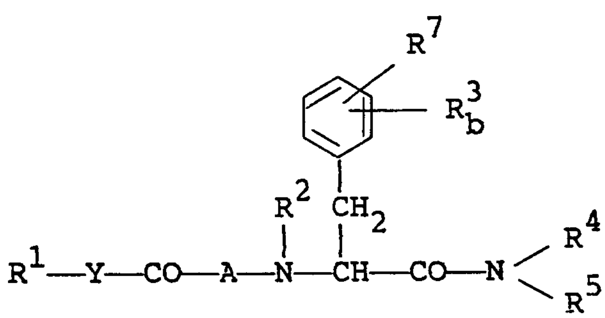

wherein $R^1$, $R^2$, $R^4$, $R^5$, $R^7$, A and Y are each as defined above, and
$R^3_b$ is carboxy($C_{1-6}$)alkoxy,
or a salt thereof, or

(3) subjecting a compound of the formula :

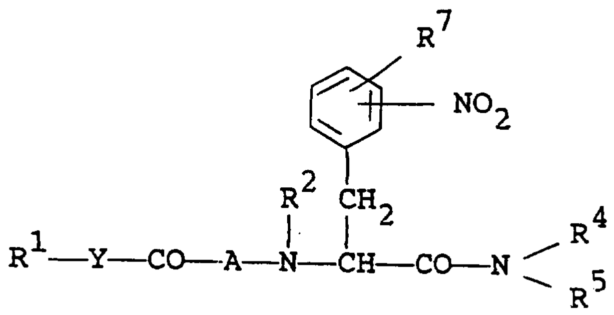

wherein $R^1$, $R^2$, $R^4$, $R^5$, $R^7$, A and Y are each as defined above, or a salt thereof, to reduction, to give a compound of the formula :

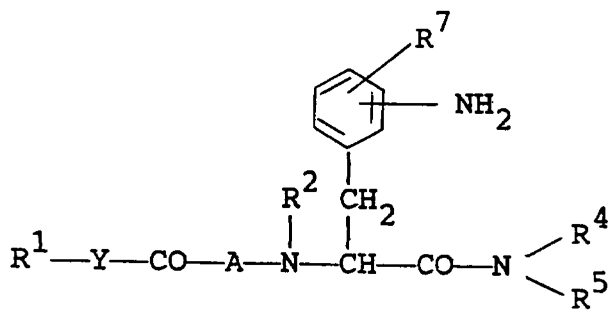

wherein $R^1$, $R^2$, $R^4$, $R^5$, $R^7$, A and Y are each as defined above, or a salt thereof.

**6.** A pharmaceutical composition which comprises a compound of claim 1 and a pharmaceutically acceptable carrier or excipient.

**7.** A process for preparing a pharmaceutical composition which comprises admixing a compound of claim 1 with a pharmaceutically acceptable carrier or excipient.

**8.** A compound of claim 1 for use as a medicament.

**9.** A compound of claim 1 for use as a tachykinin antagonist.

**10.** A compound of claim 1 for use as a substance P antagonist.

**11.** A use of a compound of claim 1 for manufacturing a medicament for treating tachykinin mediated diseases.

**Claims for the following Contracting States : ES, GR**

**1.** A process for preparing a compound of the formula :

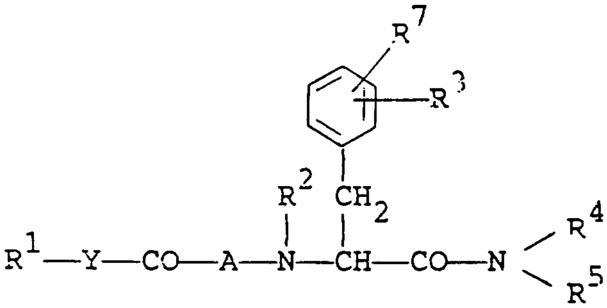

wherein

$R^1$ is $C_6$-$C_{10}$ aryl which may have one to three substituent(s) selected from halogen and $C_{1-6}$ alkoxy;
benzofuryl;
pyridyl;
or a group of the formula :

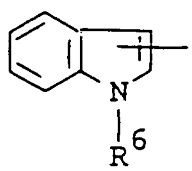

wherein $R^6$ is hydrogen; or
$C_{1-6}$ alkyl,

$R^2$ is hydrogen or $C_{1-6}$ alkyl,

$R^3$ is $C_{1-6}$ alkyl which may have one to three halogen;
amino;
acylamino;
carboxy($C_{1-6}$)alkoxy;
$C_{1-6}$ alkoxycarbonyl($C_{1-6}$)alkoxy;
halogen;
$C_{1-6}$ alkoxy; or nitro,

$R^4$ is $C_{1-6}$ alkyl,

$R^5$ is mono or di or triphenyl($C_{1-6}$)alkyl,

$R^7$ is hydrogen; or
$C_{1-6}$ alkyl;

A is hydroxyproline, and

Y is bond, $C_{1-6}$ alkylene or $C_{2-6}$ alkenylene,

or a pharmaceutically acceptable salt thereof,
which comprises

(1) reacting a compound of the formula :

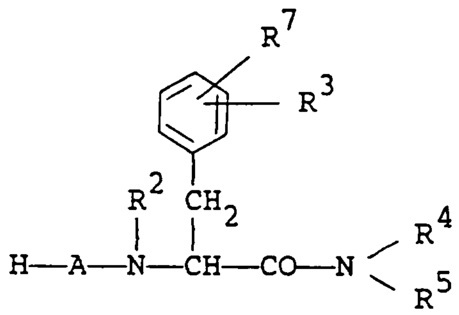

wherein $R^2$, $R^3$, $R^4$, $R^5$, $R^7$ and A are each as defined above, or its reactive derivative at the amino group or a salt thereof, with a compound of the formula :

$$R^1 - Y - COOH$$

wherein $R^1$ and Y are each as defined above, or its reactive derivative at the carboxy group or a salt thereof, to give a compound of the formula:

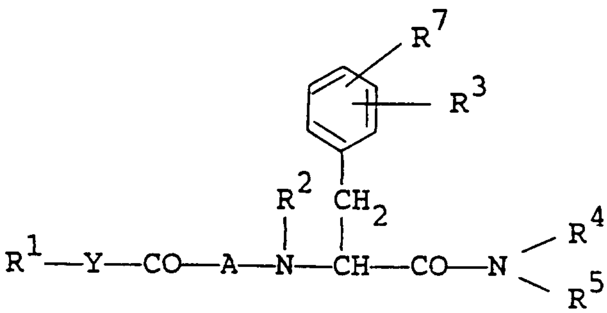

wherein $R^1$, $R^2$, $R^3$, $R^4$, $R^5$, $R^7$, A and Y are each as defined above, or a salt thereof, or

(2) subjecting a compound of the formula :

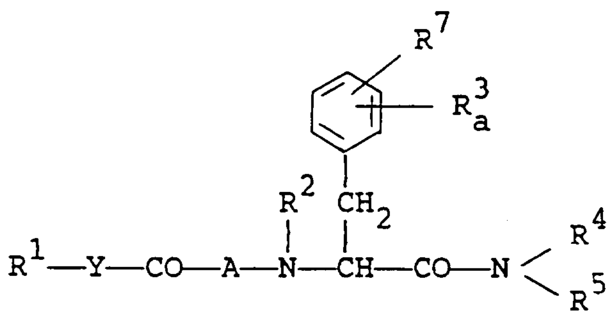

wherein $R^1$, $R^2$, $R^4$, $R^5$, A, $R^7$ and Y are each as defined above, and

$R^3_a$ is $C_{1-6}$ alkoxycarbonyl($C_{1-6}$)alkoxy,

or a salt thereof, to elimination reaction of the carboxy protective group, to give a compound of the formula :

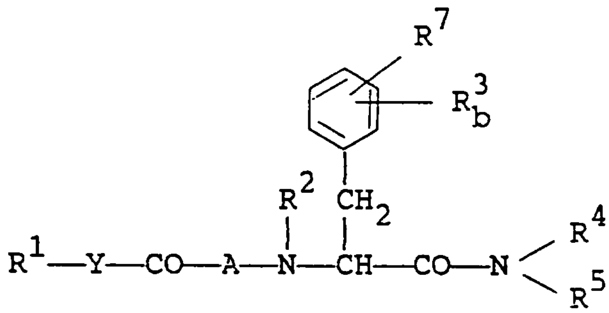

wherein $R^1$, $R^2$, $R^4$, $R^5$, $R^7$, A and Y are each as defined above, and

$R^3_b$ is carboxy($C_{1-6}$)alkoxy,

or a salt thereof, or

(3) subjecting a compound of the formula :

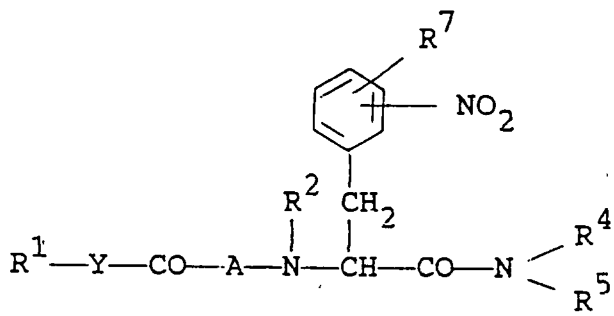

wherein $R^1$, $R^2$, $R^4$, $R^5$, $R^7$, A and Y are each as defined above, or a salt thereof, to reduction, to give a compound of the formula :

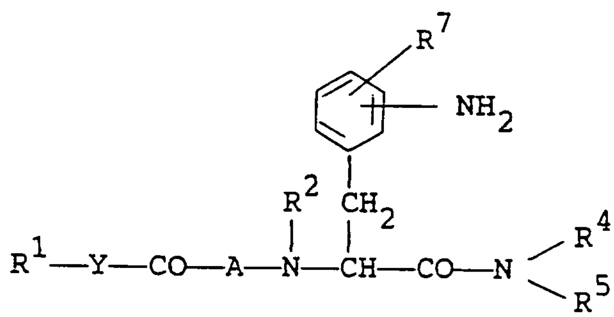

wherein $R^1$, $R^2$, $R^4$, $R^5$, $R^7$, A and Y are each as defined above, or a salt thereof.

**2.** A process according to claim 1, wherein

$R^1$ is phenyl which may have one to two halogen or one to two $C_{1-6}$ alkoxy, benzofuryl, pyridyl, indolyl or 1-$C_{1-6}$ alkylindolyl,

$R^3$ is $C_{1-6}$ alkyl, trihalo($C_{1-6}$)alkyl, amino, $C_{1-6}$ alkanesulfonylamino, carboxy($C_{1-6}$)alkoxy, $C_{1-6}$ alkoxycarbonyl($C_{1-6}$)alkoxy, halogen, $C_{1-6}$ alkoxy or nitro,

$R^5$ is phenyl($C_{1-6}$)alkyl, and

A is hydroxyproline.

**3.** A process of claim 2, wherein

| | |
|---|---|
| $R^1$ | is phenyl, difluorophenyl, dimethoxyphenyl, benzofuryl, pyridyl, indolyl, or 1-methylindolyl, |
| $R^2$ | is hydrogen or methyl, |
| $R^3$ | is methyl, trifluoromethyl, amino, methanesulfonylamino, carboxymethoxy, ethoxycarbonylmethoxy, fluoro, chloro, methoxy or nitro, |
| $R^4$ | is methyl, |
| $R^5$ | is benzyl, |
| $R^7$ | is hydrogen or methyl, and |
| Y | is bond, ethylene or vinylene. |

**4.** A process of claim 3, wherein the compound is selected from the group consisting of :

(1) 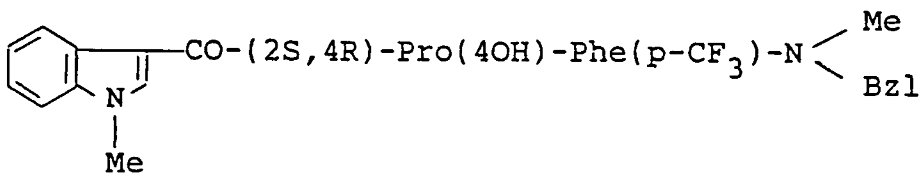

(2) 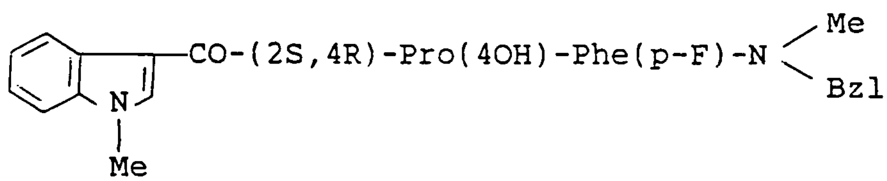

(3) 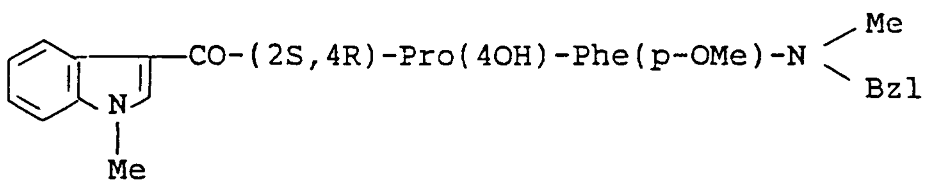

**5.** A process for preparing a pharmaceutical composition which comprises admixing a compound prepared according to claim 1 with a pharmaceutically acceptable carrier or excipient.

**Patentansprüche**

**Patentansprüche für folgende Vertragsstaaten : AT, BE, CH, DE, DK, FR, GB, IT, LI, LU, NL, SE**

**1.** Eine Verbindung der Formel:

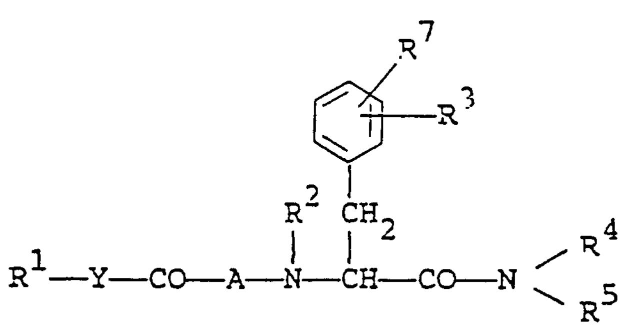

wobei

$R^1$ $C_6$-$C_{10}$-Aryl ist, der einen bis drei aus Halogen und $C_1$-$_6$-Alkoxy ausgewählte Substituent(en) aufweisen kann;
Benzofuryl;
Pyridyl;
oder eine Gruppe der Formel:

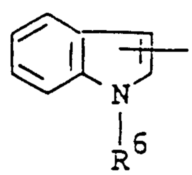

wobei $R^6$ Wasserstoff oder $C_1$-$_6$-Alkyl ist,
$R^2$ Wasserstoff oder $C_1$-$_6$-Alkyl ist,
$R^3$ $C_1$-$_6$-Alkyl ist, der ein bis drei Halogen aufweisen kann;
Amino;
Acylamino;
Carboxy($C_1$-$_6$)alkoxy;
$C_1$-$_6$-Alkoxycarbonyl($C_1$-$_6$)alkoxy;
Halogen;
$C_1$-$_6$-Alkoxy oder Nitro;
$R^4$ $C_1$-$_6$-Alkyl ist,
$R^5$ Mono- oder Di- oder Triphenyl($C_1$-$_6$)alkyl ist,
$R^7$ Wasserstoff oder $C_1$-$_6$-Alkyl ist;
A Hydroxyprolin ist, und
Y eine Bindung, $C_1$-$_6$-Alkylen oder $C_2$-$_6$-Alkenylen ist,

und ein pharmazeutisch verträgliches Salz davon.

**2.** Eine Verbindung aus Anspruch 1, wobei

$R^1$ Phenyl ist, der ein bis zwei Halogen oder ein bis zwei $C_1$-$_6$-Alkoxy aufweisen kann, Benzofuryl, Pyridyl, Indolyl oder 1-$C_1$-$_6$-Alkylindolyl,
$R^3$ $C_1$-$_6$-Alkyl, Trihalo($C_1$-$_6$)alkyl, Amino, $C_1$-$_6$-Alkansulfonylamino, Carboxy($C_1$-$_6$)alkoxy, $C_1$-$_6$-Alkoxycarbonyl($C_1$-$_6$)alkoxy, Halogen, C1-$_6$-Alkoxy oder Nitro ist,
$R^5$ Phenyl($C_1$-$_6$)alkyl ist, und
A Hydroxyprolin ist.

**3.** Eine Verbindung aus Anspruch 2, wobei

$R^1$ Phenyl, Difluorphenyl, Dimethoxyphenyl, Benzofuryl, Pyridyl, Indolyl oder 1-Methylindolyl ist,
$R^2$ Wasserstoff oder Methyl ist,
$R^3$ Methyl, Trifluormethyl, Amino, Methansulfonylamino, Carboxymethoxy, Ethoxycarbonylmethoxy, Fluor, Chlor, Methoxy oder Nitro ist,
$R^4$ Methyl ist,
$R^5$ Benzyl ist,
$R^7$ Wasserstoff oder Methyl ist, und
Y eine Bindung, Ethylen oder Vinylen ist.

**4.** Eine Verbindung aus Anspruch 3, die ausgewählt wird aus der Gruppe, bestehend aus:

(1) 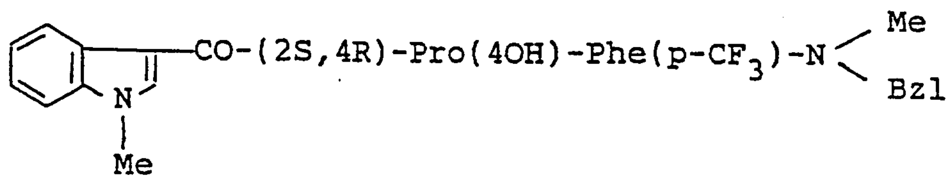

(2) 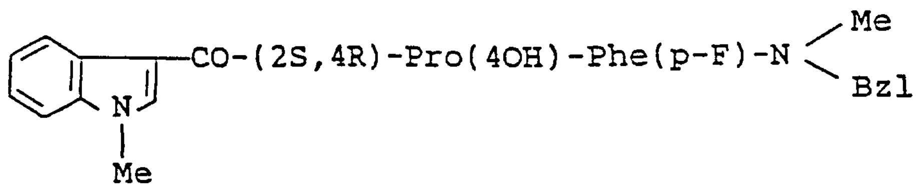

(3) 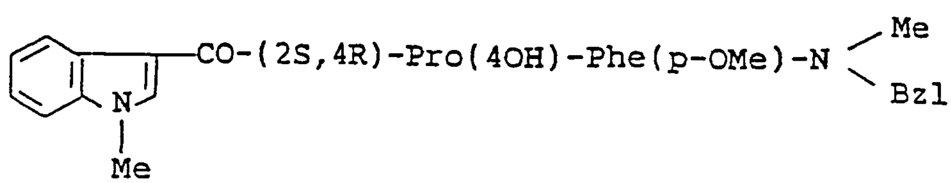

5. Ein Verfahren zur Herstellung einer Verbindung aus Anspruch 1, oder eines pharmazeutisch verträglichen Salzes davon, welches umfaßt

(1) Umsetzen einer Verbindung der Formel:

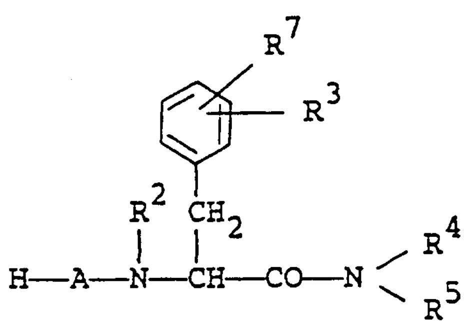

wobei $R^2$, $R^3$, $R^4$, $R^5$, $R^7$ und A wie oben definiert sind,
oder ihres reaktiven Derivats an der Aminogruppe oder eines Salzes davon mit einer Verbindung der Formel:

$$R^1 - Y - COOH$$

wobei $R^1$ und Y wie oben definiert sind, oder ihres reaktiven Derivates an der Carboxygruppe oder eines Salzes davon zur Herstellung einer Verbindung der Formel:

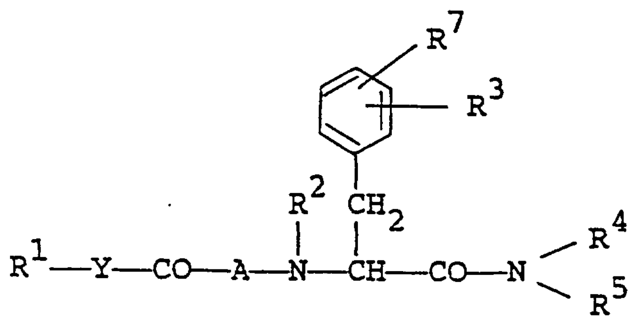

wobei $R^1$, $R^2$, $R^3$, $R^4$, $R^5$, $R^7$, A und Y jeweils wie oben definiert sind, oder eines Salzes davon, oder

(2) Durchführen einer Eliminierungsreaktion der Carboxyschutzgruppe mit einer Verbindung der Formel:

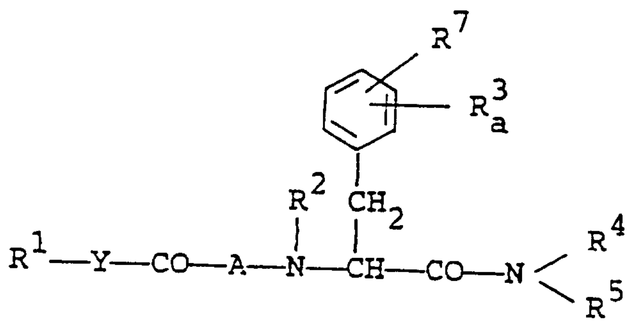

wobei $R^1$, $R^2$, $R^4$, $R^5$, A, $R^7$ und Y jeweils wie oben definiert sind, und
$R_a^3$ $C_1$-$_6$-Alkoxycarbonyl($C_1$-$_6$)alkoxy ist,
oder eines Salzes davon, zur Herstellung einer Verbindung der Formel:

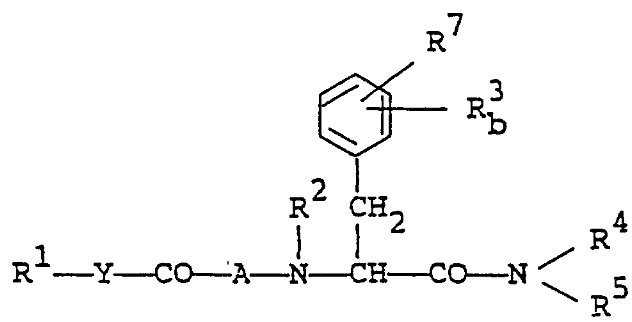

wobei $R^1$, $R^2$, $R^4$, $R^5$, $R^7$, A und Y jeweils wie oben definiert sind, und
$R_b^3$ Carboxy($C_1$-$_6$)alkoxy ist,
oder eines Salzes davon, oder

(3) Durchführung einer Reduktion mit einer Verbindung der Formel:

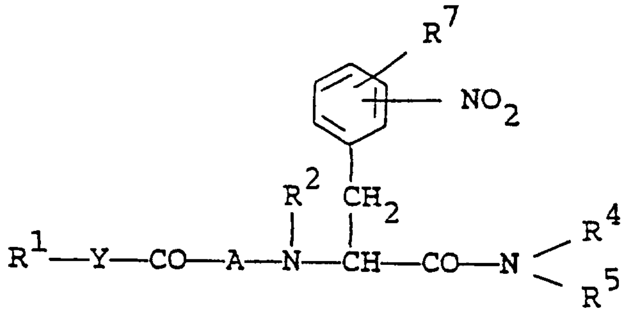

wobei $R^1$, $R^2$, $R^4$, $R^5$, $R^7$, A und Y jeweils wie oben definiert sind, oder eines Salzes davon, zur Herstellung einer Verbindung der Formel:

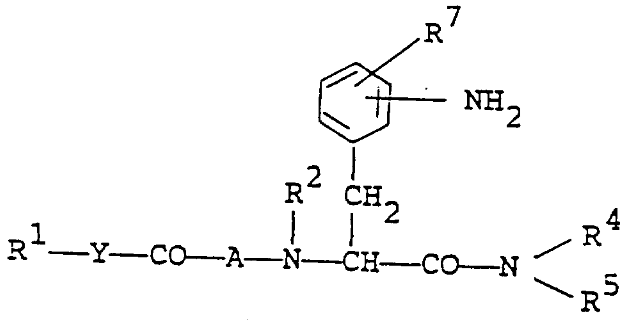

wobei $R^1$, $R^2$, $R^4$, $R^5$, $R^7$, A und Y jeweils wie oben definiert sind, oder eines Salzes davon.

6. Eine pharmazeutische Zusammensetzung, die eine Verbindung aus Anspruch 1 und einen pharmazeutisch verträglichen Träger oder Exzipienten umfaßt.

7. Ein Verfahren zur Herstellung einer pharmazeutischen Zusammensetzung, welches Mischen einer Verbindung aus Anspruch 1 mit einem pharmazeutisch verträglichen Träger oder Excipienten umfaßt.

8. Eine Verbindung aus Anspruch 1 zur Verwendung als Medikament.

9. Eine Verbindung aus Anspruch 1 zur Verwendung als Tachykinin-Antagonist.

10. Eine Verbindung aus Anspruch 1 zur Verwendung als Substanz P-Antagonist.

11. Verwendung einer Verbindung aus Anspruch 1 zur Herstellung eines Medikamentes zur Behandlung von Tachykinin-vermittelten Erkrankungen.

**Patentansprüche für folgende Vertragsstaaten : ES, GR**

1. Ein Verfahren zur Herstellung einer Verbindung der Formel:

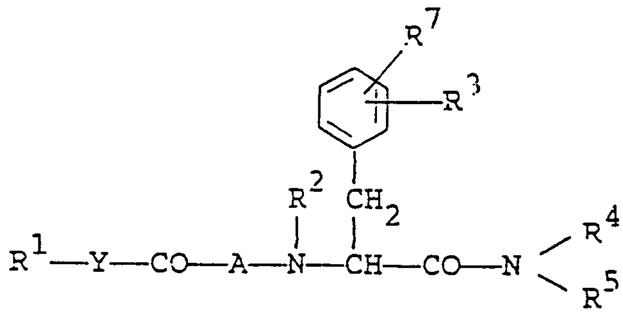

wobei

$R^1$ $C_6$-$C_{10}$-Aryl ist, der einen bis drei aus Halogen und $C_{1}$-$_{6}$-Alkoxy ausgewählte Substituent(en) aufweisen kann;
Benzofuryl;
Pyridyl;
oder eine Gruppe der Formel:

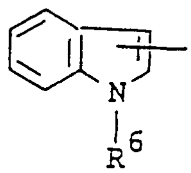

ist, wobei $R^6$ Wasserstoff oder $C_1$-$_6$-Alkyl ist,
$R^2$ Wasserstoff oder $C_1$-$_6$-Alkyl ist,
$R^3$ $C_1$-$_6$-Alkyl ist, der ein bis drei Halogen aufweisen kann;
Amino;
Acylamino;
Carboxy($C_1$-$_6$)alkoxy;
$C_1$-$_6$-Alkoxycarbonyl($C_1$-$_6$)alkoxy;
Halogen;
$C_1$-$_6$-Alkoxy oder Nitro;
$R^4$ $C_1$-$_6$-Alkyl ist,
$R^5$ Mono- oder Di- oder Triphenyl($C_1$-$_6$)alkyl ist,
$R^7$ Wasserstoff oder $C_1$-$_6$-Alkyl ist;
A Hydroxyprolin ist, und
Y eine Bindung, $C_1$-$_6$-Alkylen oder $C_2$-$_6$-Alkenylen ist,

oder eines pharmazeutisch verträglichen Salzes davon, welches umfaßt

(1) Umsetzung einer Verbindung der Formel:

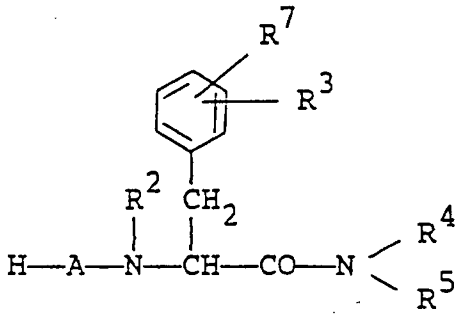

wobei $R^2$, $R^3$, $R^4$, $R^5$, $R^7$ und A wie oben definiert sind,
oder ihres reaktiven Derivats an der Aminogruppe oder eines Salzes davon mit einer Verbindung der Formel:

$$R^1 - Y - COOH$$

wobei $R^1$ und Y wie oben definiert sind, oder ihres reaktiven Derivates an der Carboxygruppe oder eines Salzes davon, zur Herstellung einer Verbindung der Formel:

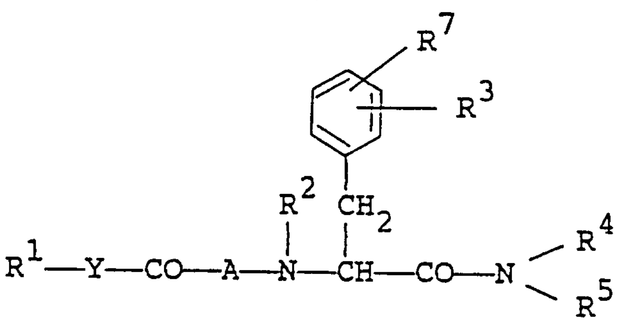

wobei $R^1$, $R^2$, $R^3$, $R^4$, $R^5$, $R^7$, A und Y jeweils wie oben definiert sind,
oder eines Salzes davon, oder

(2) Durchführen einer Eliminierungsreaktion der Carboxyschutzgruppe mit einer Verbindung der Formel:

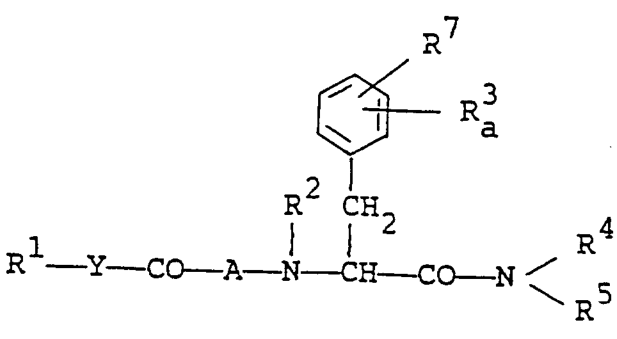

wobei $R^1$, $R^2$, $R^4$, $R^5$, A, $R^7$ und Y jeweils wie oben definiert sind, und
$R_a^3$ $C_1$-$_6$-Alkoxycarbonyl($C_1$-$_6$)alkoxy ist,
oder eines Salzes davon, zur Herstellung einer Verbindung der Formel:

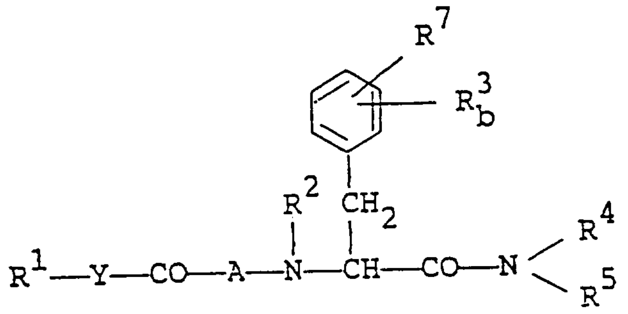

wobei $R^1$, $R^2$, $R^4$, $R^5$, $R^7$, A und Y jeweils wie oben definiert sind, und
$R_b^3$ Carboxy($C_1$-$_6$)alkoxy ist,
oder eines Salzes davon, oder

(3) Durchführung einer Reduktion mit einer Verbindung der Formel:

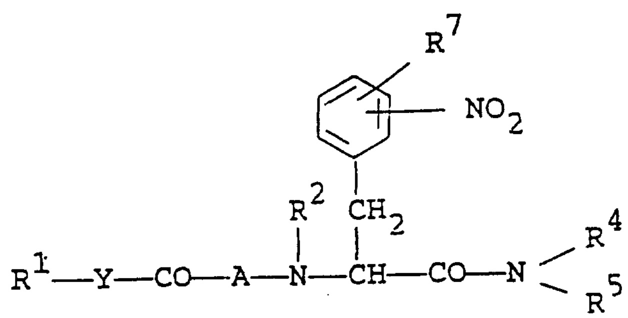

wobei $R^1$, $R^2$, $R^4$, $R^5$, $R^7$, A und Y jeweils wie oben definiert sind,
oder eines Salzes davon, zur Herstellung einer Verbindung der Formel:

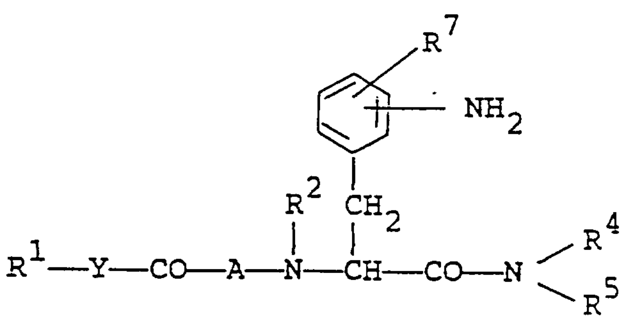

wobei $R^1$, $R^2$, $R^4$, $R^5$, $R^7$, A und Y jeweils wie oben definiert sind,
oder eines Salzes davon.

**2.** Ein Verfahren entsprechend Anspruch 1, wobei

$R^1$ Phenyl ist, der ein bis zwei Halogene oder ein bis zwei $C_1$-$_6$-Alkoxy aufweisen kann, Benzofuryl, Pyridyl, Indolyl oder 1-$C_1$-$_6$-Alkylindolyl
$R^3$ $C_1$-$_6$-Alkyl, Trihalo($C_1$-$_6$)alkyl, Amino, $C_1$-$_6$-Alkansulfonylamino, Carboxy($C_1$-$_6$)alkoxy, $C_1$-$_6$-Alkoxycarbonyl($C_1$-$_6$)alkoxy, Halogen, $C_1$-$_6$-Alkoxy oder Nitro ist,
$R^5$ Phenyl($C_1$-$_6$)alkyl ist, und
A Hydroxyprolin ist.

3. Ein Verfahren nach Anspruch 2, wobei

$R^1$ Phenyl, Difluorphenyl, Dimethoxyphenyl, Benzofuryl, Pyridyl, Indolyl oder 1-Methylindolyl ist,
$R^2$ Wasserstoff oder Methyl ist,
$R^3$ Methyl, Trifluormethyl, Amino, Methansulfonylamino, Carboxymethoxy, Ethoxycarbonylmethoxy, Fluor, Chlor, Methoxy oder Nitro ist,
$R^4$ Methyl ist,
$R^5$ Benzyl ist,
$R^7$ Wasserstoff oder Methyl ist, und
Y eine Bindung, Ethylen oder Vinylen ist.

4. Ein Verfahren nach Anspruch 3, wobei die Verbindung ausgewählt wird aus der Gruppe, bestehend aus:

(1) 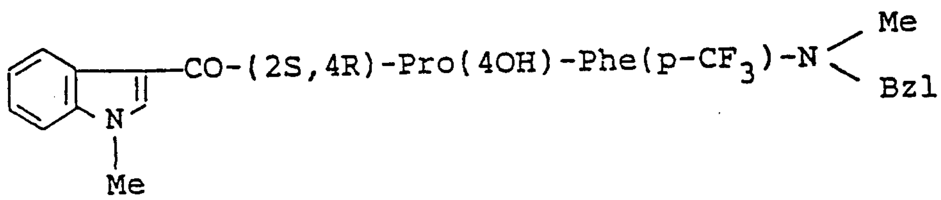

(2) 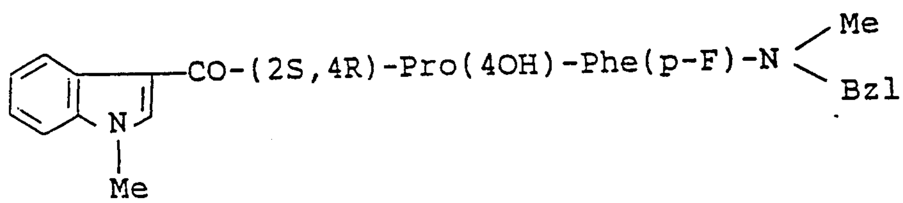

(3) 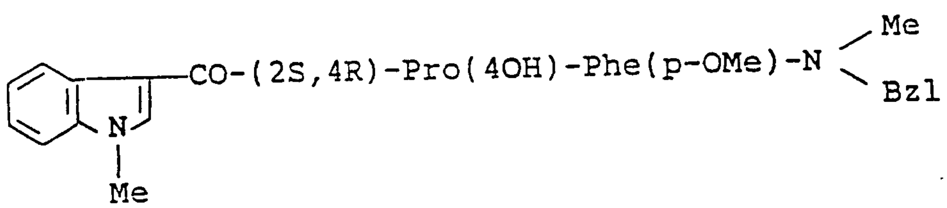

5. Ein Verfahren zur Herstellung einer pharmazeutischen Zusammensetzung, welches Mischen einer nach Anspruch 1 hergestellten Verbindung mit einem pharmazeutisch verträglichen Träger oder Exzipienten umfaßt.

**Revendications**

**Revendications pour les Etats contractants suivants : AT, BE, CH, DE, DK, FR, GB, IT, LI, LU, NL, SE**

1. Composé de la formule :

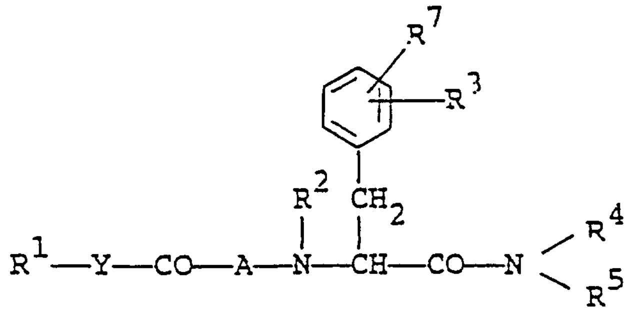

dans laquelle

$R^1$ est un groupe aryle en $C_6$-$C_{10}$ qui peut avoir un à trois substituants choisis parmi un atome d'halogène et un groupe alcoxy en $C_1$-$C_6$;
un groupe benzofuryle,
un groupe pyridyle;
ou un groupe de la formule :

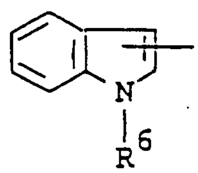

dans laquelle

$R^6$ est un atome d'hydrogène; ou
un groupe alkyle en $C_1$-$C_6$,
$R^2$ est un atome d'hydrogène ou un groupe alkyle en $C_1$-$C_6$,
$R^3$ est un groupe alkyle en $C_1$-$C_6$ qui peut avoir un à trois atomes d'halogène;
un groupe amino;
un groupe acylamino;
un groupe carboxy(alcoxy en $C_1$-$C_6$);
un groupe (alcoxy en $C_1$-$C_6$)carbonyl(alcoxy en $C_1$-$C_6$);
un atome d'halogène;
un groupe alcoxy en $C_1$-$C_6$; ou un groupe nitro,
$R^4$ est un groupe alkyle en $C_1$-$C_6$;
$R^5$ est un groupe mono ou di ou triphényl(alkyle en $C_1$-$C_6$),
$R^7$ est un atome d'hydrogène; ou
un groupe alkyle en $C_1$-$C_6$;
A est un groupe hydroxyproline, et
Y est une liaison, un groupe alkylène en $C_1$-$C_6$ ou un groupe alcénylène en $C_2$-$C_6$.

et un sel pharmaceutiquement acceptable de celui-ci.

2. Composé selon la revendication 1, dans lequel :

$R^1$ est un groupe phényle qui peut avoir un à deux atomes d'halogène ou un à deux groupes alcoxy en $C_1$-$C_6$, benzofuryle, pyridyle, indolyle ou 1-(alkyle en $C_1$-$C_6$)indolyle,
$R^3$ est un groupe alkyle en $C_1$-$C_6$, trihalo(alkyle en $C_1$-$C_6$), amino, (alcane en $C_1$-$C_6$)sulfonylamino, carboxy(alcoxy en $C_1$-$C_6$), (alcoxy en $C_1$-$C_6$)carbonyl(alcoxy en $C_1$-$C_6$), un atome d'halogène, un groupe

alcoxy en $C_1$-$C_6$ ou nitro,

$R^5$ est un groupe phényl(alkyle en $C_1$-$C_6$), et

A est un groupe hydroxyproline.

**3.** Composé selon la revendication 2, dans lequel :

$R^1$ est un groupe phényle, difluorophényle, diméthoxyphényle, benzofuryle, pyridyle, indolyle ou 1-méthylindolyle,

$R^2$ est un atome d'hydrogène ou un groupe méthyle,

$R^3$ est un groupe méthyle, trifluorométhyle, amino, méthanesulfonylamino, carboxyméthoxy, éthoxycarbonylméthoxy, fluoro, chloro, méthoxy ou nitro,

$R^4$ est un groupe méthyle,

$R^5$ est un groupe benzyle,

$R^7$ est un atome d'hydrogène ou un groupe méthyle, et

Y est une liaison, un groupe éthylène ou vinylène.

**4.** Composé selon la revendication 3, qui est choisi dans le groupe constitué de :

(1) 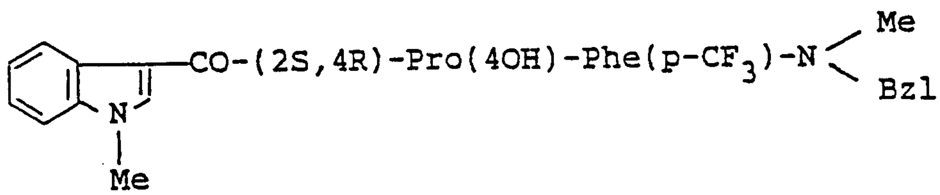

(2) 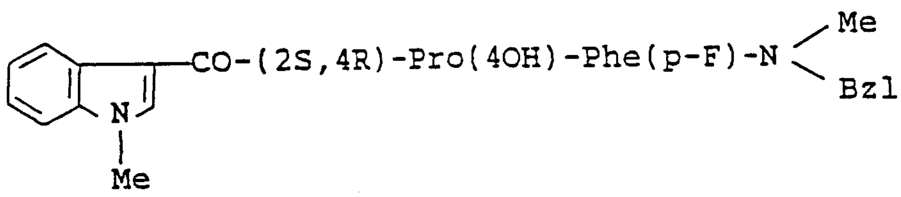

(3) 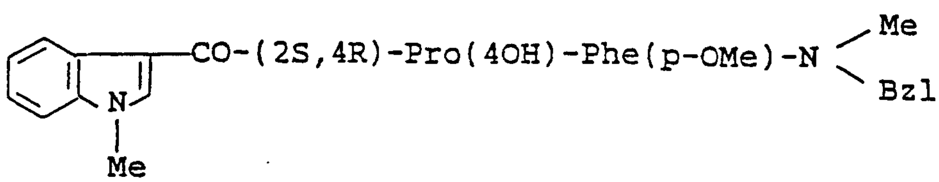

**5.** Procédé pour préparer un composé de la revendication 1, ou un de ses sels pharmaceutiquement acceptables, qui comprend :

(1) la mise à réagir d'un composé de la formule :

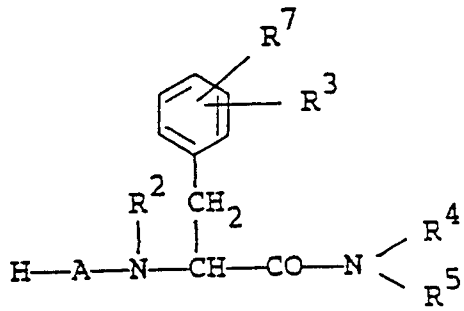

dans laquelle $R^2$, $R^3$, $R^4$, $R^5$, $R^7$ et A sont chacun tels que définis ci-dessus,
ou son dérivé réactif au groupe amino,
ou un de ses sels, avec un composé de la formule :

$$R^1 - Y - COOH$$

dans laquelle $R^1$ et Y sont chacun tels que définis ci-dessus,
ou son dérivé réactif au groupe carboxy,
ou un de ses sels, pour obtenir un composé de la formule :

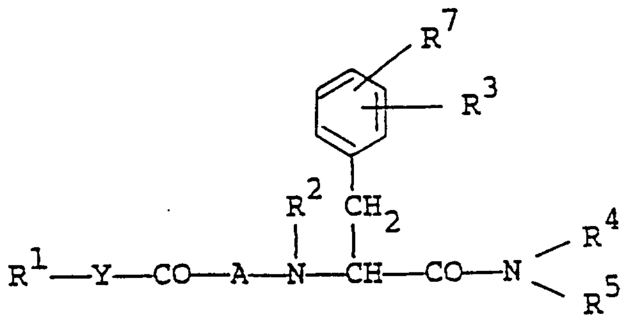

dans laquelle $R^1$, $R^2$, $R^3$, $R^4$, $R^5$, $R^7$, A et Y sont chacun tels que définis ci-dessus,
ou un de ses sels, ou
(2) la soumission d'un composé de la formule :

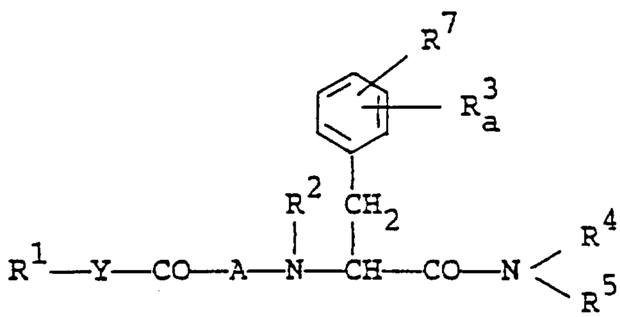

dans laquelle $R^1$, $R^2$, $R^4$, $R^5$, A, $R^7$ et Y sont chacun tels que définis ci-dessus, et
$R^3_a$ est un groupe (alcoxy en $C_1$-$C_6$)carbonyl(alcoxy en $C_1$-$C_6$),
ou un de ses sels, à une réaction d'élimination du groupe protecteur du groupe carboxy, pour obtenir un composé de la formule :

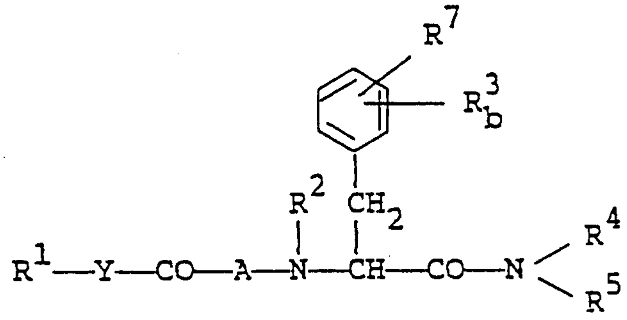

dans laquelle $R^1$, $R^2$, $R^4$, $R^5$, $R^7$, A et Y sont chacun tels que définis ci-dessus, et $R^3_b$ est un groupe carboxy(alcoxy en $C_1$-$C_6$), ou un de ses sels, ou

(3) la soumission d'un composé de la formule :

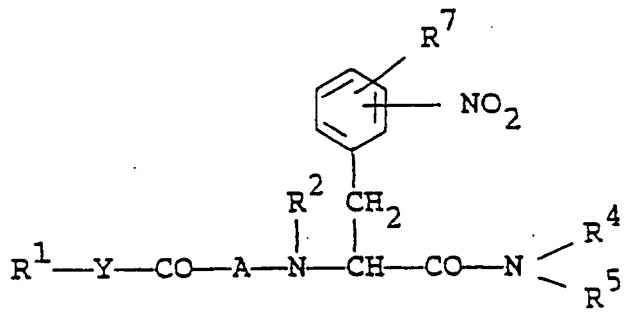

dans laquelle $R^1$, $R^2$, $R^4$, $R^5$, $R^7$, A et Y sont chacun tels que définis ci-dessus, ou un de ses sels, à une réduction, pour donner un composé de la formule :

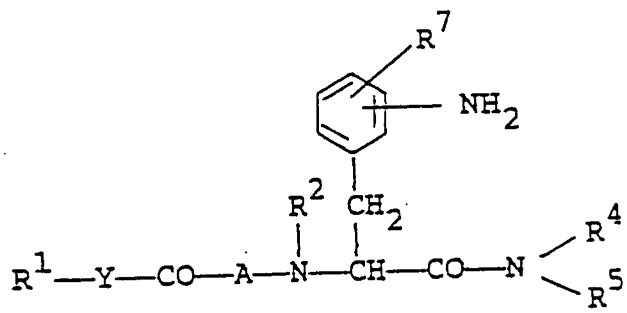

dans laquelle $R^1$, $R^2$, $R^4$, $R^5$, $R^7$, A et Y sont chacun tels que définis ci-dessus, ou un de ses sels.

**6.** Composition pharmaceutique qui comprend un composé de la revendication 1 et un support ou excipient pharmaceutiquement acceptables.

**7.** Procédé pour préparer une composition pharmaceutique qui comprend le mélange d'un composé de la revendication 1 avec un support ou excipient pharmaceutiquement acceptables.

**8.** Composé selon la revendication 1, pour utilisation comme médicament.

**9.** Composé selon la revendication 1 pour emploi comme antagoniste de la tachykinine.

**10.** Composé selon la revendication 1 pour emploi comme antagoniste de la substance P.

**11.** Utilisation d'un composé de la revendication 1 pour la fabrication d'un médicament pour le traitement des maladies soulagées par la tachykinine.

**Revendications pour les Etats contractants suivants : ES, GR**

**1.** Procédé pour préparer un composé de la formule :

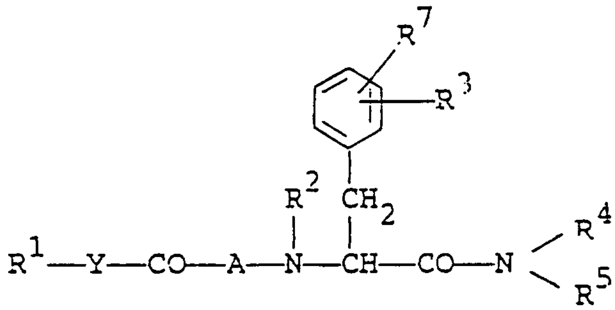

dans laquelle

$R^1$ est un groupe aryle en $C_6$-$C_{10}$ qui peut avoir un à trois substituants choisis parmi un atome d'halogène et un groupe alcoxy en $C_1$-$C_6$;
un groupe benzofuryle,
un groupe pyridyle;
ou un groupe de la formule :

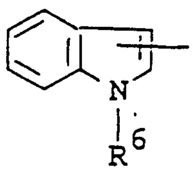

dans laquelle

$R^6$ est un atome d'hydrogène; ou
un groupe alkyle en $C_1$-$C_6$,
$R^2$ est un atome d'hydrogène ou un groupe alkyle en $C_1$-$C_6$,
$R^3$ est un groupe alkyle en $C_1$-$C_6$ qui peut avoir un à trois atomes d'halogène;
un groupe amino;
un groupe acylamino;
un groupe carboxy(alcoxy en $C_1$-$C_6$);
un groupe (alcoxy en $C_1$-$C_6$)carbonyl(alcoxy en $C_1$-$C_6$);
un atome d'halogène;
un groupe alcoxy en $C_1$-$C_6$; ou un groupe nitro,
$R^4$ est un groupe alkyle en $C_1$-$C_6$;
$R^5$ est un groupe mono ou di ou triphényl(alkyle en $C_1$-$C_6$),
$R^7$ est un atome d'hydrogène; ou
un groupe alkyle en $C_1$-$C_6$;
A est un groupe hydroxyproline, et
Y est une liaison, un groupe alkylène en $C_1$-$C_6$ ou un groupe alcénylène en $C_2$-$C_6$.

ou un de ses sels pharmaceutiquement acceptables qui comprend :

(1) la mise à réagir d'un composé de la formule :

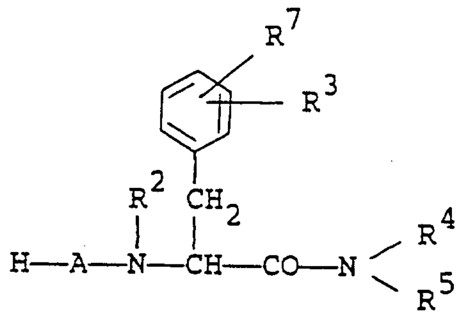

dans laquelle $R^2$, $R^3$, $R^4$, $R^5$, $R^7$ et A sont chacun tels que définis ci-dessus, ou son dérivé réactif au groupe amino, ou un de ses sels, avec un composé de la formule :

$$R^1 - Y - COOH$$

dans laquelle $R^1$ et Y sont chacun tels que définis ci-dessus, ou son dérivé réactif au groupe carboxy, ou un de ses sels, pour donner un composé de la formule :

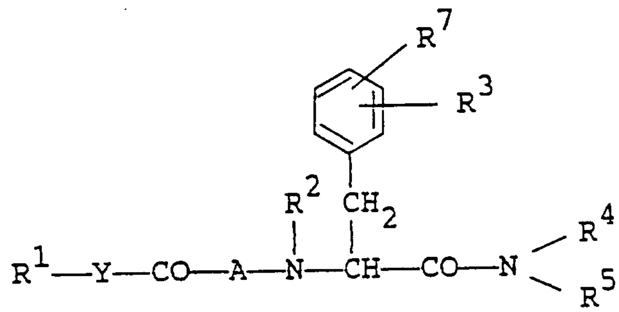

dans laquelle $R^1$, $R^2$, $R^3$, $R^4$, $R^5$, $R^7$, A et Y sont chacun tels que définis ci-dessus, ou un de ses sels, ou

(2) la soumission d'un composé de la formule :

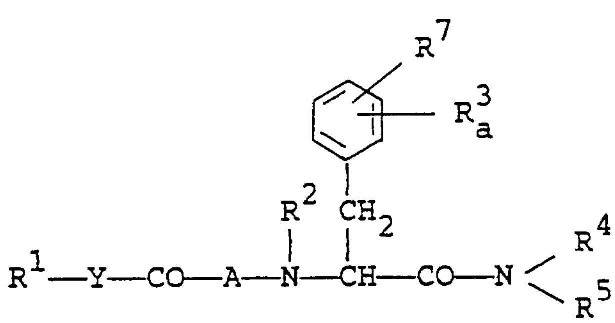

dans laquelle $R^1$, $R^2$, $R^4$, $R^5$, A, $R^7$ et Y sont chacun tels que définis ci-dessus, et $R^3_a$ est un groupe (alcoxy en $C_1$-$C_6$)carbonyl(alcoxy en $C_1$-$C_6$), ou un de ses sels, à une réaction d'élimination du groupe protecteur du groupe carboxy, pour donner un composé de la formule :

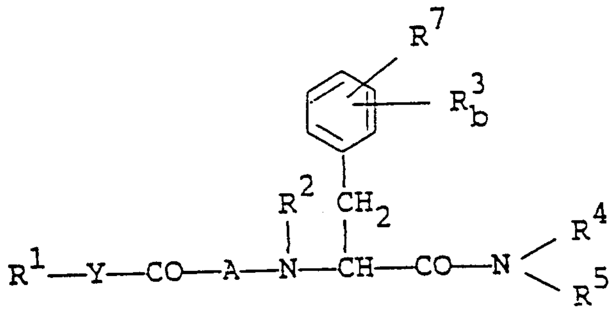

dans laquelle $R^1$, $R^2$, $R^4$, $R^5$, $R^7$, A et Y sont chacun tels que définis ci-dessus, et
$R^3_b$ est un groupe carboxy(alcoxy en $C_1$-$C_6$),
ou un de ses sels, ou
(3) la soumission d'un composé de la formule :

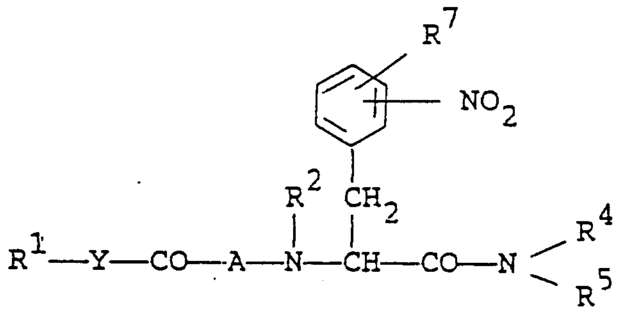

dans laquelle $R^1$, $R^2$, $R^4$, $R^5$, $R^7$, A et Y sont chacun tels que définis ci-dessus, ou un de ses sels, à une réduction, pour donner un composé de la formule :

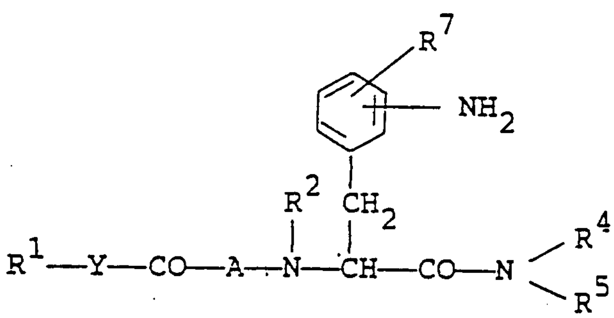

dans laquelle $R^1$, $R^2$, $R^4$, $R^5$, $R^7$, A et Y sont chacun tels que définis ci-dessus, ou un de ses sels.

**2.** Procédé selon la revendication 1, dans lequel :

$R^1$ est un groupe phényle qui peut avoir un à deux atomes d'halogène ou un à deux groupes alcoxy en $C_1$-$C_6$, benzofuryle, pyridyle, indolyle ou 1-(alkyle en $C_1$-$C_6$)indolyle,
$R^3$ est un groupe alkyle en $C_1$-$C_6$, trihalo(alkyle en $C_1$-$C_6$), amino, (alcane en $C_1$-$C_6$)sulfonylamino, carboxy(alcoxy en $C_1$-$C_6$), (alcoxy en $C_1$-$C_6$)carbonyl(alcoxy en $C_1$-$C_6$), un atome d'halogène, un groupe alcoxy en $C_1$-$C_6$ ou un groupe nitro,
$R^5$ est un groupe phényl(alkyle en $C_1$-$C_6$), et
A est un groupe hydroxyproline.

**3.** Procédé selon la revendication 2, dans lequel :

| | |
|---|---|
| $R^1$ | est un groupe phényle, difluorophényle, diméthoxyphényle, benzofuryle, pyridyle, indolyle ou 1-méthylindolyle, |
| $R^2$ | est un atome d'hydrogène ou un groupe méthyle, |
| $R^3$ | est un groupe méthyle, trifluorométhyle, amino, méthanesulfonylamino, carboxyméthoxy, éthoxycarbonylméthoxy, fluoro, chloro, méthoxy ou nitro, |
| $R^4$ | est un groupe méthyle, |
| $R^5$ | est un groupe benzyle, |
| $R^7$ | est un atome d'hydrogène ou un groupe méthyle, et |
| Y | est une liaison, un groupe éthylène ou vinylène. |

**4.** Procédé selon la revendication 3, dans lequel le composé est choisi dans le groupe constitué de:

(1) 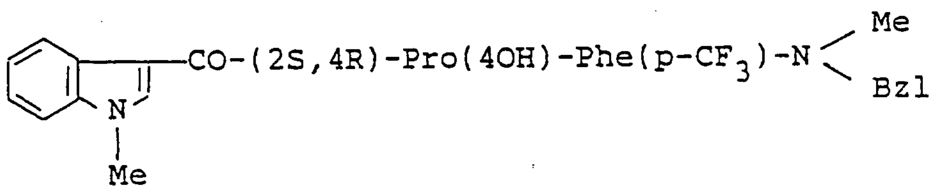

(2) 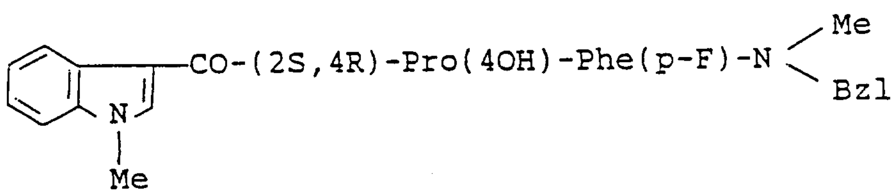

(3) 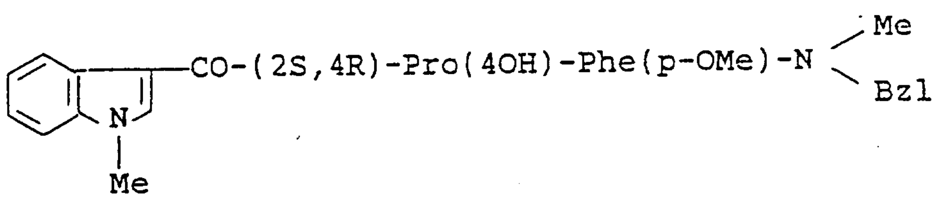

**5.** Procédé pour préparer une composition pharmaceutique qui comprend le mélange d'un composé préparé conformément à la revendication 1 avec un support ou excipient pharmaceutiquement acceptables.